(19) Europäisches Patentamt
European Patent Office
Office européen des brevets

(11) Publication number: **0 272 866**
**A1**

(12) # EUROPEAN PATENT APPLICATION

(21) Application number: **87311030.8**

(22) Date of filing: **15.12.87**

(51) Int. Cl.[4]: **C 07 D 487/04**
A 61 K 31/55
//(C07D487/04,257:00,243:00),
(C07D487/04,249:00,243:00),
(C07D487/04,243:00,235:00),
(C07D487/04,243:00,231:00),
(C07D487/04,243:00,209:00)

(30) Priority: **23.12.86 US 945583**

(43) Date of publication of application:
**29.06.88 Bulletin 88/26**

(84) Designated Contracting States:
**CH DE FR GB IT LI NL**

(71) Applicant: **MERCK & CO. INC.**
**126, East Lincoln Avenue P.O. Box 2000**
**Rahway New Jersey 07065-0900 (US)**

(72) Inventor: **Freidinger, Roger M.**
**2185 Rebecca Drive**
**Hatfield Pennsylvania 19440 (US)**

**Bock, Mark G.**
**1603 Leon Drive**
**Hatfield Pennsylvania 19440 (US)**

**Evans, Ben E.**
**501 Perkiomen Avenue**
**Lansdale Pennsylvania 19446 (US)**

(74) Representative: **Hesketh, Alan, Dr.**
**European Patent Department Merck & Co., Inc. Terlings Park Eastwick Road**
**Harlow Essex, CM20 2QR (GB)**

(54) **1,4-Benzodiazepines with 5-membered heterocyclic rings.**

(57) Aromatic 1,4-benzodiazepines with fused 5-membered heterocyclic rings which are antagonists of cholecystokinins and/or gastrin, and are useful in the treatment or prevention of CCK-related and/or gastrin-related disorders of the gastrointestinal, central nervous and appetite regulatory systems; compositions comprising these compounds; and methods of treatment in mammals and of increasing food intake of animals employing these compounds.

EP 0 272 866 A1

## Description

# 1,4-BENZODIAZEPINES WITH 5-MEMBERED HETEROCYCLIC RINGS

## BACKGROUND OF THE INVENTION

Cholecystokinins (CCK) and gastrin are structurally-related neuropeptides which exist in gastrointestinal tissue and in the the central nervous system (see, V. Mutt, Gastrointestinal Hormones, G. B. J. Glass, Ed., Raven Press, N.Y., p. 169 and G. Nisson, ibid, p. 127).

Cholecystokinins include CCK-33, a neuropeptide of thirty-three amino acids in its originally isolated form (see, Mutt and Jorpes, Biochem J. 125, 678 (1971)), its carboxylterminal octapeptide, CCK-8 (a naturally-occurring neuropeptide, also, and the minimum fully active sequence), and 39- and 12-amino acid forms, while gastrin occurs in 34-, 17- and 14-amino acid forms, with the minimum active sequence being the C-terminal pentapeptide, Gly-Trp-Met-Asp-Phe-$NH_2$, which is the common structural element shared by both CCK and gastrin.

CCK's are believed to be physiological satiety hormones, thereby possibly playing an important role in appetite regulation (G. P. Smith, Eating and Its Disorders, A.J. Stunkard and E. Stellar, Eds, Raven Press, New York, 1984, p. 67), as well as also stimulating colonic motility, gall bladder contraction, pancreatic enzyme secretion, and inhibiting gastric emptying. They reportedly co-exist with dopamine in certain mid-brain neurons and thus may also play a role in the functioning of dopaminergic systems in the brain, in addition to serving as neurotransmitters in their own right (see: A. J. Prange et al., "Peptides in the Central Nervous System", Ann. Repts. Med. Chem. 17, 31, 33 [1982] and references cited therein; J. A. Williams, Biomed. Res. 3 107 [1982]); and J. E Morley, Life Sci. 30, 479, [1982]).

The primary role of gastrin, on the other hand, appears to be stimulation of the secretion of water and electrolytes from the stomach, and, as such, it is involved in control of gastric acid and pepsin secretion. Other physiological effects of gastrin then include increased mucosal blood flow and increased antral motility, with rat studies having shown that gastrin has a positive trophic effect on the gastric mucosa, as evidenced by increased DNA, RNA and protein synthesis.

Antagonists to CCK and to gastrin have been useful for preventing and treating CCK-related and/or gastrin-related disorders of the gastrointestinal (GI) and central nervous (CNS) systems of animals, especially of humans. Just as there is some overlap in the biological activities of CCK and gastrin, antagonists also tend to have affinity for both receptors. In a practical sense, however, there is enough selectivity to the different receptors that greater activity against specific CCK- or gastrin-related disorders can often also be identified.

Selective CCK antagonists are themselves useful in treating CCK-related disorders of the appetite regulatory systems of animals as well as in potentiating and prolonging opiate-mediated analgesia, thus having utility in the treatment of pain [see P. L. Faris et al., Science 226, 1215 (1984)], while selective gastrin antagonists are useful in the modulation of CNS behavior, as a palliative for gastrointestinal neoplasms, and in the treatment and prevention of gastrin-related disorders of the gastrointestinal system in humans and animals, such as peptic ulcers, Zollinger-Ellison syndrome, antral G cell hyperplasia and other conditions in which reduced gastrin activity is of therapeutic value.

Also, since CCK and gastrin also have trophic effects on certain tumors [K. Okyama, Hokkaido J. Med. Sci., 60, 206-216 (1985)], antagonists of CCK and gastrin are useful in treating these tumors [see, R.D. Beauchamp et al., Ann. Surg., 202,303 (1985)].

Four distinct chemical classes of CCK-receptor antagonists have been reported. The first class comprises derivatives of cyclic nucleotides, of which dibutyryl cyclic GMP has been shown to be the most potent by detailed structure-function studies (see, N. Barlos et al., Am. J. Physiol., 242, G 161 (1982) and P. Robberecht et al., Mol., Pharmacol., 17, 268 (1980)).

The second class comprises peptide antagonists which are C-terminal fragments and analogs of CCK, of which both shorter (Boc-Met-Asp-Phe-$NH_2$, Met-Asp-Phe-$NH_2$), and longer (Cbz-Tyr($SO_3H$)-Met-Gly-Trp-Met-Asp-$NH_2$) C-terminal fragments of CCK can function as CCK antagonists, according to recent structure-function studies (see, R. T. Jensen et al., Biochem. Biophys. Acta., 757, 250 (1983), and M. Spanarkel et al., J. Biol. Chem., 258, 6746 (1983)). The latter compound was recently reported to be a partial agonist [see, J. M. Howard et al., Gastroenterology 86(5) Part 2, 1118 (1984)].

Then, the third class of CCK-receptor antagonists comprises the amino acid derivatives: proglumide, a derivative of glutaramic acid, and the N-acyl tryptophans including para-chlorobenzoyl-L-tryptophan (benzotript), [see, W. F. Hahne et al., Proc. Natl. Acad. Sci. U.S.A., 78, 6304 (1981), R. T. Jensen et al., Biochem. Biophys. Acta., 761, 269 (1983)]. All of these compounds, however, are relatively weak antagonists of CCK ($IC_{50}$: generally $10^{-4}$M[although more potent analogs of proglumide have been recently reported in F. Makovec et al., Arzneim-Forsch Drug Res., 35 (II) (1985) and in German Patent Application DE 3522506A1], but down to $10^{-6}$M in the case of peptides), and the peptide CCK-antagonists have substantial stability and absorption problems.

In addition, a fourth class consists of improved CCK-antagonists comprising a nonpeptide of novel structure from fermentation sources [R. S. L. Chang et al., Science, 230, 177-179 (1985)] and 3-substituted benzodiazepines based on this structure [published European Patent Applications 167 919, 167 920 and 169 392, B. E. Evans et al, Proc. Natl. Acad. Sci. U.S.A., 83, p. 4918-4922 (1986) and R.S.L. Chang et al, ibid,

p. 4923-4926] have also been reported.

No really effective receptor antagonists of the in vivo effects of gastrin have been reported (J. S. Morley, Gut Pept. Ulcer Proc., Hiroshima Symp. 2nd, 1983, p. 1), and very weak in vitro antagonists, such as proglumide and certain peptides have been described [(J. Martinez, J. Med. Chem. 27, 1597 (1984)]. Recently, however, pseudopeptide analogs of tetragastrin have been reported to be more effective gastrin antagonists than previous agents [J. Martinez et al., J. Med. Chem., 28, 1874-1879 (1985)].

The benzodiazepine (BZD) structure class, which has been widely exploited as therapeutic agents, especially as central nervous system (CNS) drugs, such as anxiolytics, and which exhibits strong binding to "benzodiazepine receptors" in vitro, has not in the past been reported to bind to CCK or gastrin receptors. Benzodiazepines have been shown to antagonize CCK-induced activation of rat hippocampal neurones but this effect is mediated by the benzodiazepine receptor, not the CCK receptor [see J. Bradwejn et al., Nature, 312, 363 (1984)]. Of these reported BZD's, additionally, the large majority do not contain substituents attached to the 3-position of the seven membered ring, as it is well known in the art that 3-substituents result in decreasing anxiolitic activity, especially as these substituents increase in size. Further, it has been demonstrated that in the case of the 3-substituted benzodiazepines that have been reported, the preferred stereochemistry at position 3 for CNS activity is S, which would correspond to an L-amino acid, such as L-tryptophan.

It was, therefore, an abject of this invention to identify substances which more effectively antagonize the function of cholecystokinins and/or gastrin in disease states in mammals, especially in humans. It was another object of this invention to prepare novel compounds which more selectively inhibit cholecystokinins or inhibit gastrin. It was still another object of this invention to develop a method of antagonizing the functions of cholecystokinin and/or gastrin in disease states in mammals. It is also an object of this invention to develop a method of preventing or treating disorders of the gastrointestinal, central nervous and appetite regulatory systems of mammals, especially of humans, or of increasing food intake of animals.

## SUMMARY OF THE INVENTION

The present invention is directed to aromatic 1,4-benzodiazepines with fused 5-membered heterocyclic rings which are antagonists of cholecystokinins (CCK) and/or gastrin, and are useful in the treatment and prevention of CCK-related and/or gastrin-related disorders of the gastrointestinal, central nervous and appetite regulatory systems of mammals, especially of humans, including irritable bowel syndrome, ulcers, excess pancreatic or gastric secretion, acute pancreatis, motility disorders, neuroleptic disorder, tardive dyskinesia, Parkinson's disease, psychosis, Gilles de la Tourette Syndrome, disorders of the appetite regulatory system, Zollinger-Ellison syndrome, antral G cell hyperplasia, pain (by the potentiation of opioid analgesics), and malignancies of the lower esophagus, stomach, intestines, and colon. As antagonists of CCK, they may also be used to increase food intake of animals.

## DETAILED DESCRIPTION OF THE INVENTION

The 1,4-benzodiazepines with fused 5-membered heterocyclic rings of this invention are those of Formula I:

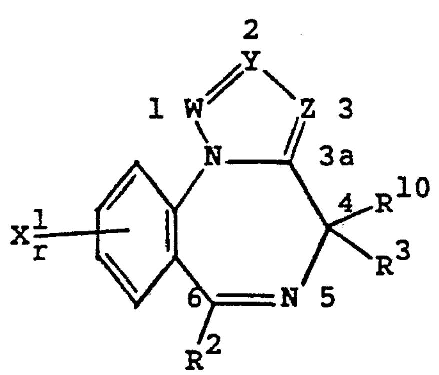

(I),

wherein

$R^1$ is H, $C_1$-$C_4$-straight- or branched-chain alkyl, cyclo-$C_3$-$C_7$-alkyl, or unsubstituted or mono- or disubstituted phenyl (where the substituent(s) is/are selected from the group consisting of halo, $C_1$-$C_4$-alkyl, $C_1$-$C_4$-alkoxy, and hydroxy);

$R^2$ is H, $C_1$-$C_4$-straight- or branched-chain alkyl, mono- or disubstituted or unsubstituted phenyl (where the substituent(s) is/are selected from the group consisting of halo, $C_1$-$C_4$-alkyl, $C_1$-$C_4$-alkoxy, $C_1$-$C_4$-alkylthio, carboxyl, carboxyl-$C_1$-$C_4$-alkyl, nitro, $-CF_3$,

$O\overset{O}{\overset{\|}{C}}-R^4$ and hydroxy), 2-, 3- or 4-pyridyl or $-(CH_2)_mCOOR^6$;

$R^3$ is 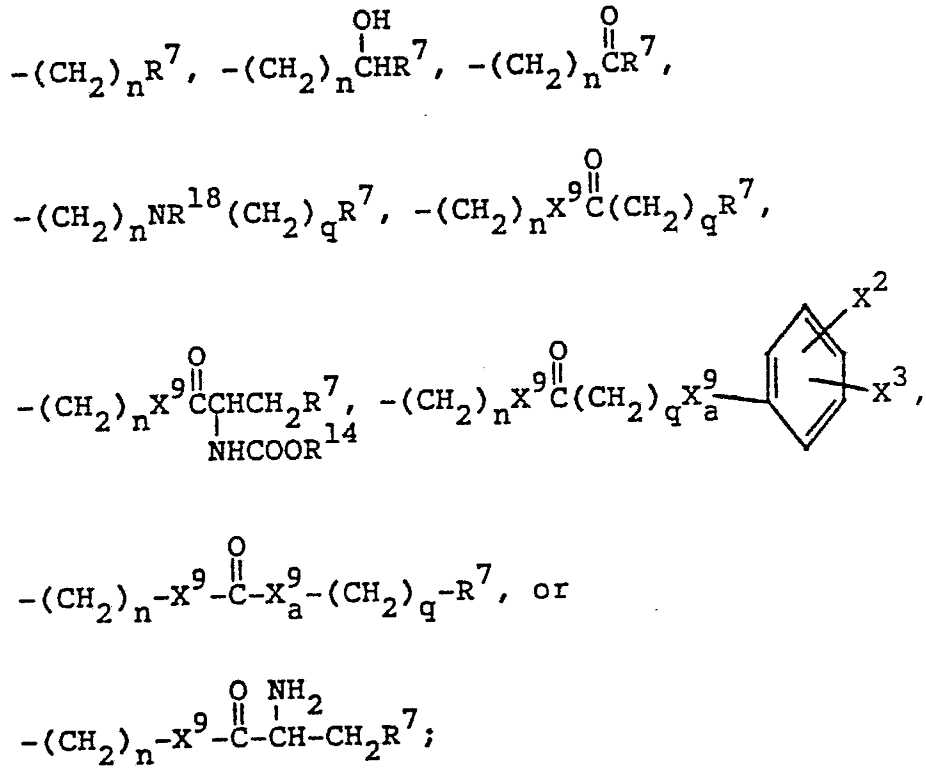

$R^4$ and $R^5$ are independently H, $C_1$-$C_4$-straight- or branched-chain-alkyl, cyclo-$C_3$-$C_7$-alkyl, or are connected to form a hetero ring of the form,- $N(CH_2)_k$, where k is 2 to 6;

$R^6$ is H, $C_1$-$C_4$-straight or branched-chain alkyl, cyclo-$C_3$-$C_5$-alkyl, unsubstituted or mono- or disubstituted phenyl (where the substituent(s) is/are selected from the group consisting of halo, $C_1$-$C_4$-alkyl, $C_1$-$C_4$-alkoxy, nitro, and $CF_3$), or unsubstituted or mono- or disubstituted phenyl-$C_1$-$C_4$-straight or branched-chain alkyl (where the substituent(s) is/are selected from the group consisting of halo, $C_1$-$C_4$-alkyl, $C_1$-$C_4$-alkoxy, nitro, and $CF_3$);

$R^7$ is α- or β-naphthyl, unsubstituted or mono- or disubstituted phenyl (where the substituent(s) is/are selected from the group consisting of halo, $-NO_2$, -OH, $-NR^4R^5$, $C_1$-$C_4$-straight- or branched-chain alkyl, cyano, phenyl, trifluoromethyl, acetylamino, acetyloxy, $C_1$-$C_4$-straight- or branched-chain alkylthio, $SCF_3$, $C\equiv CH$, $CH_2SCF_3$, $OCHF_2$, S-phenyl, or $C_1$-$C_4$-straight- or branched-chain alkoxy),

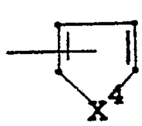 , 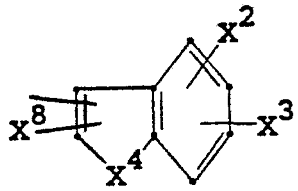 , 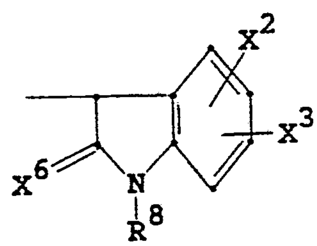

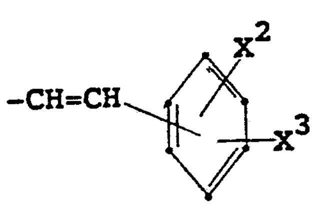 or 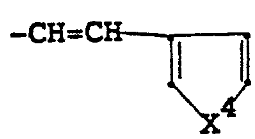 ;

$R^8$ is H, $C_1$-$C_4$-straight- or branched-chain alkyl, cyclo-$C_3$-$C_7$-alkyl, $-(CH_2)_n$-cyclo-$C_3$-$C_7$-alkyl,

$-\overset{O}{\overset{\|}{C}}-C_1-C_4$-straight- or branched-chain alkyl, or

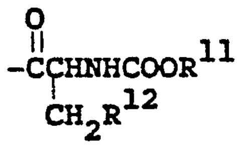

;

$R^9$ is OH, $OR^{11}$ or

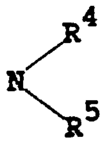

;

$R^{10}$ is H, -OH, or $-CH_3$;
$R^{11}$ and $R^{12}$ are independently $C_1$-$C_4$-straight- or branched-chain alkyl or cyclo-$C_3$-$C_7$-alkyl;
$R^{13}$ is H or $C_1$-$C_4$-straight- or branched-chain alkyl, cyclo-$C_3$-$C_7$-alkyl,

cyclo-$C_3$-$C_7$-alkyl, $(CH_2)_n\overset{O}{\overset{\|}{C}}R^9$, $(CH_2)_mO\overset{O}{\overset{\|}{C}}R^{11}$,

$(CH_2)_mNHR^{15}$, $(CH_2)_mOH$, or phenyl-$C_1$-$C_4$-straight- or branched-chain alkyl;
$R^{14}$ is $C_1$-$C_4$-straight- or branched-chain alkyl or phenyl-$C_1$-$C_4$-straight- or branched-chain alkyl;
$R^{15}$ is H or $\overset{O}{\overset{\|}{C}}-R^{11}$;
$R^{16}$ is H, $C_1$-$C_4$-straight- or branched-chain alkyl, cyclo-$C_3$-$C_7$-alkyl, or phenyl-$C_1$-$C_4$-straight- or branched-chain alkyl;
$R^{17}$ is H, $C_1$-$C_4$-straight- or branched-chain alkyl, $COOR^{16}$, phenyl or phenyl-$C_1$-$C_4$-straight- or branched-chain alkyl;
$R^{18}$ H, $C_1$-$C_4$-straight- or branched-chain alkyl or formyl, acetyl, propionyl or butyryl;
m is 1-to-4;
n is 0-to-4;
q id 0-to-4;
r is 1 or 2;
$X^1$ is H, $-NO_2$, $CF_3$, CN, OH, $C_1$-$C_4$-straight- or branched-chain alkyl, halo, $C_1$-$C_4$-straight- or branched-chain alkylthio, $C_1$-$C_4$-straight- or branched-chain alkoxy,
$-(CH_2)_nCOOR^6$, $-NR^4R^5$, or $O-\overset{O}{\overset{\|}{C}}-R^4$;
$X^2$ and $X^3$ are independently H, -OH, $-NO_2$, halo, $C_1$-$C_4$-straight- or branched-chain alkylthio, $C_1$-$C_4$-straight- or branched-chain alkyl, $C_1$-$C_4$-straight- or branched-chain alkoxy, or
$-O-\overset{O}{\overset{\|}{C}}-R^4$;
$X^4$ is S, O, $CH_2$, or $NR^8$;
$X^6$ is O or HH;
$X^8$ is H or $C_1$-$C_4$-straight- or branched-chain alkyl;
$X^9$ and $X^9_a$ are independently $NR^{18}$ or O;
W = $CR^1$ or N;
Y = $CR^{13}$ or N; and
Z = N or $CR^{17}$,
and the pharmaceutically-acceptable salts thereof.

As used herein, the definition of each expression, i.e., m, n, p, $C_1$-$C_4$-alkyl, etc., when it occurs more than once in any structure, is intended to be independent of its definition elsewhere in the same structure.

In the compounds of Formula I, the preferred stereochemistry for CCK-antagonism relates to D-tryptophan, where $C^{3a}$ and $N^5$ of Formula I correspond to the carbonyl carbon and α-amino nitrogen of D-tryptophan and $R^3$ occupies the position of the indolylmethyl side chain.

In the compounds of Formula I, the preferred stereochemistry for gastrin antagonism may be either D or L depending on the nature of $R^3$. For example,

when $R^3 = (CH_2)_nR^7$ or $(CH_2)_nX^9\overset{O}{\overset{\|}{C}}(CH_2)_qR^7$, the preferred stereochemistry corresponds to D-tryptophan, as above. When $R^3 = (CH_2)_nX^9\overset{O}{\overset{\|}{C}}X^9_a(CH_2)_qR^7$, the preferred stereochemistry corresponds to L-tryptophan.

As used herein, halo is F, Cl, Br, or I; and $C_1$-$C_4$-alkyl includes methyl, ethyl, propyl, isopropyl, butyl, isobutyl, and t-butyl.

Preferred compounds according to the instant invention are those wherein $R^1$ is H or $C_1$-$C_4$-alkyl; $R^2$ is unsubstituted or mono- or disubstituted phenyl (wherein the substituents are selected from the group consisting of halo, $C_1$-$C_4$-alkyl, $C_1$-$C_4$-alkoxy, nitro and $-CF_3$); $R^3$ is $-(CH_2)_nR^7$,

$-(CH_2)_nX^9\overset{O}{\overset{\|}{C}}(CH_2)_qR^7$ or $-(CH_2)_n-X^9-\overset{O}{\overset{\|}{C}}-X^9_a-(CH_2)_q-R^7$; $R^4$ and $R^5$ are independently H, $C_1$-$C_4$-alkyl or are connected to form the ring $-\overline{N(CH_2)_k}$, where k is 4 or 5; $R^7$ is α- or β-naphthyl, unsubstituted or mono- or disubstituted phenyl (wherein the substituents are selected from the group consisting of halo, $-NO_2$, $C_1$-$C_4$-alkyl, $C_1$-$C_4$-alkoxy and trifluoromethyl),

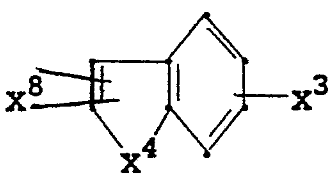 , or -CH=CH 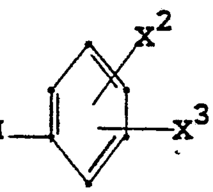 ;

$R^8$ is H or $C_1$-$C_4$-alkyl; $R^{11}$ is $C_1$-$C_4$-alkyl; $R^{13}$ is H, $C_1$-$C_4$-alkyl, cyclo-$C_3$-$C_7$-alkyl or

$-C_4$-alkyl; $R^{13}$ is H,

$(CH_2)_n\overset{O}{\overset{\|}{C}}-R^9$;

$R^{16}$ is $C_1$-$C_4$-alkyl; n is 0-to-2; q is 0-to-2; $X^1$ is H, $-NO_2$, $C_1$-$C_4$-alkyl, halo or $C_1$-$C_4$-alkoxy; $X^2$ and $X^3$ are independently H, $-NO_2$, halo, $C_1$-$C_4$-alkyl or $C_1$-$C_4$-alkoxy; and $X^9$ and $X^9_a$ are independently NH or O.

Particularly preferred compounds include those wherein $R^1$ is H or methyl; $R^2$ is phenyl or o-F-phenyl; $R^3$ is $NH\overset{O}{\overset{\|}{C}}-R^7$ or $NH\overset{O}{\overset{\|}{C}}NH-R^7$; $R^7$ is

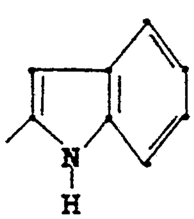 , 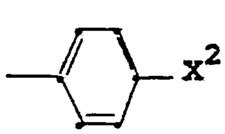 , 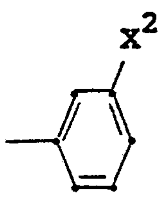 , or 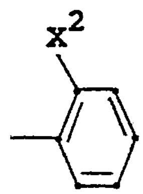 ;

$R^{13}$ is H, methyl, ethyl or propyl; $R^{17}$ is H, methyl or ethyl; $X^1$ is H; $X^2$ is H, $-NO_2$, halo, methyl or methoxy; W is $CR^1$; Y is $CR^{13}$ and Z is $CR^{17}$. Other particularly preferred compounds include those wherein $R^1$ is H or methyl;

$R^2$ is phenyl or o-F-phenyl;

$R^3$ is $-NH\overset{\overset{O}{\|}}{C}-R^7$ or $NH\overset{\overset{O}{\|}}{C}NH-R^7$; $R^7$ is 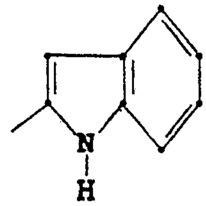 ,

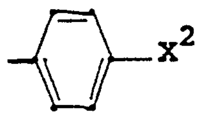 , 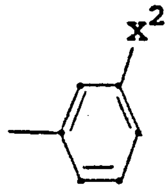 , or 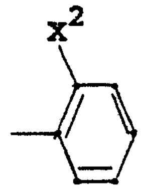 ; $R^{13}$ is H;

$X^1$ is H; $X^2$ is H, $-NO_2$, halo, methyl or methoxy; W is $CR^1$; Y is $CR^{13}$; and Z is N. Still other particularly preferred compounds include those wherein $R^1$ is H or methyl; $R^2$ is phenyl or o-F-phenyl; $R^3$ is $NH\overset{\overset{O}{\|}}{C}-R^7$ or $NH\overset{\overset{O}{\|}}{C}NH-R^7$; $R^7$ is 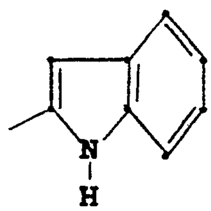 , 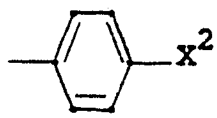 , 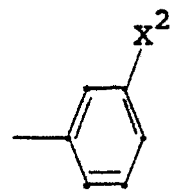 ,

or 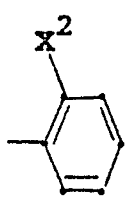 ;

$R^{17}$ is H, $CH_3$, $CH_2CH_3$, COOH, $COOCH_3$, or $COOCH_2CH_3$; $X^1$ is H; $X^2$ is H, $-NO_2$, halo, methyl, or methoxy; W is $CR^1$; Y is N and Z is $CR^{17}$. Yet other particularly preferred compounds include those wherein $R^2$ is phenyl or o-F-phenyl; $R^3$ is $NH\overset{\overset{O}{\|}}{C}-R^7$ or $NH\overset{\overset{O}{\|}}{C}NH-R^7$; $R^4$ and $R^5$ are independently H, methyl, ethyl or

$-(CH_2)_4-$; $R^7$ is 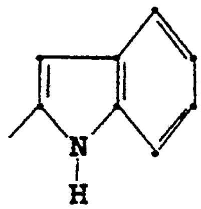 , 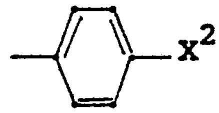 , 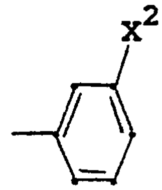 ,

or 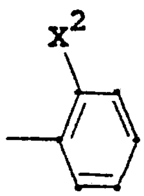 ; $R^9$ is OH, $OCH_3$, $OCH_2CH_3$, or 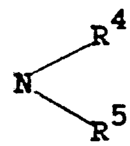 ;

$R^{13}$ is methyl, ethyl, propyl or $\overset{O}{\overset{\|}{C}}-R^9$; $R^{17}$ is H; $X^1$ is H; $X^2$ is H, $-NO_2$, halo, methyl, or methoxy; W is N; Y is $CR^{13}$; and Z is $CR^{17}$. Then, other particularly preferred compounds include those wherein $R^2$ is phenyl or o-F-phenyl; $R^3$ is $NH\overset{O}{\overset{\|}{C}}-R^7$ or $NH\overset{O}{\overset{\|}{C}}NH-R^7$; $R^7$ is

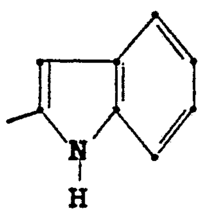 , 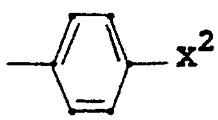 , 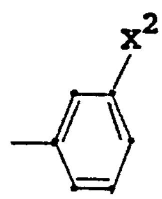 , or 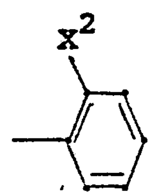 ;

$R^{17}$ is H, methyl, $COOCH_3$, or $COOCH_2CH_3$; $X^1$ is H; $X^2$ is H, $-NO_2$, halo, methyl or methoxy; W is N; Y is N; Z is $CR^{17}$. Still other particularly preferred compounds include those wherein $R^2$ is phenyl or o-F-phenyl; $R^3$ is $NH\overset{O}{\overset{\|}{C}}-R^7$ or $NH\overset{O}{\overset{\|}{C}}NH-R^7$; $R^7$ is 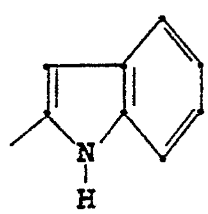 ,

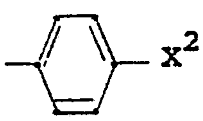 , 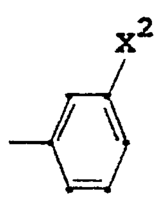 , or 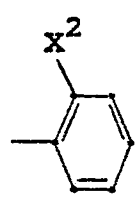 ;

$X^1$ is H; $X^2$ is H, $-NO_2$, halo, methyl or methoxy; W is N; Y is N; and Z is N.

Even more particularly preferred compounds include, for CCK antagonism:

4(S)-4(2-indolecarbonylamino)-6-phenyl-4H-pyrrolo[1,2-a]-1,4-benzodiazepine;

4(S)-4(4-chlorophenylcarbonylamino)-6-phenyl-4H-imidazo-[1,2-a]-1,4-benzodiazepine;

4(S)-4-(2-indolecarbonylamino)-1-methyl-6-phenyl-3-propyl-4H-imidazo-[1,5-a]1,4-benzodiazepine;
4(S)-4-(2-indolecarbonylamino)-2-methyl-6-phenyl-4H-pyrazolo[1,5-a]-1,4-benzodiazepine;
4(S)-4-(2-indolecarbonylamino)-3-methyl-6-phenyl-4H-triazolo-[1,5-a]-1,4-benzodiazepine; and
4(S)-4-(2-indolecarbonylamino)-6-phenyl-4H-tetrazolo-[1,5-a]-1,4-benzodiazepine;
and for gastrin antagonism:
4(R)-4(3-methoxyphenylaminocarbonylamino)-6-phenyl-4H-pyrrolo[1,2-a]-1,4-benzodiazepine;
4(R)-4-(3-methylphenylaminocarbonylamino)-6-phenyl-4H-imidazo-[1,2-a]-1,4-benzodiazepine;
4(R)-4-(3-chlorophenylaminocarbonylamino)-1-methyl-6-phenyl-3-propyl-4H-imidazo-[1,2-a]-1,4-benzodiazepine;
4(R)-4-(3-methoxyphenylaminocarbonylamino)-6-phenyl-4H-pyrazolo-[1,5-a]-1,4-benzodiazepine-2-carboxylic acid, ethyl ester;
4(R)-4-(3-methylphenylaminocarbonylamino)-3-methyl-6-phenyl-4H-triazolo-[1,5-a]-1,4-benzodiazepine; and
4(R)-4-(3-chlorophenylaminocarbonylamino)-6-phenyl-4H-tetrazolo-[1,5-a]-1,4-benzodiazepine.

The pharmaceutically-acceptable salts of the compounds of Formula I include the conventional non-toxic salts or the quarternary ammonium salts of the compounds of Formula I formed, e.g., from non-toxic inorganic or organic acids. For example, such conventional non-toxic salts include those derived from inorganic acids such as hydrochloric, hydrobromic, sulfuric, phosphoric, nitric and the like; and the salts prepared from organic acids such as acetic, propionic, succinic, glycolic, stearic, lactic, malic, tartaric, citric, pamoic, maleic, hydroxymaleic, phenylacetic, glutamic, benzoic, salicylic, sulfanilic, 2-acetoxy-benzoic, fumaric, toluenesulfonic, methanesulfonic, ethane disulfonic, isethionic, and the like.

The compounds of Formula I are particularly distinguished from benzodiazepines of the prior art by the presence of 3-substituents. These Formula I compounds bind strongly to CCK-receptors, but only weakly to benzodiazepine-receptors, especially with the increasing size of the 3-substituent.

Compounds according to Formula I may be prepared according to Schemes I through XI as follows:

SCHEME I

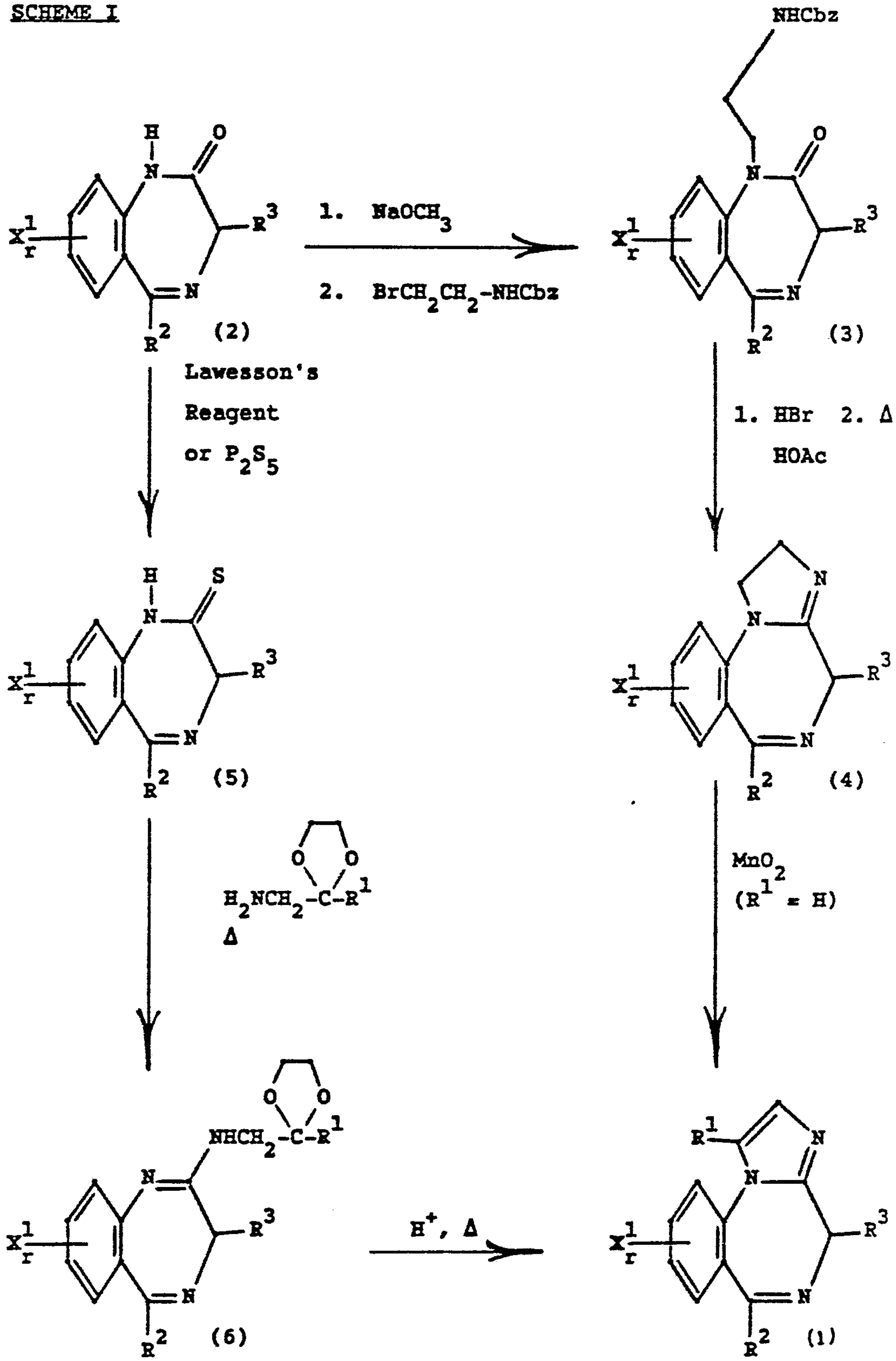
1. $NaOCH_3$
2. $BrCH_2CH_2$-NHCbz
(2)
(3)
Lawesson's Reagent or $P_2S_5$
1. HBr 2. Δ
HOAc
(5)
(4)
$H_2NCH_2$-C-$R^1$
Δ
$MnO_2$
($R^1$ = H)
(6)
$H^+$, Δ
(1)

SCHEME II

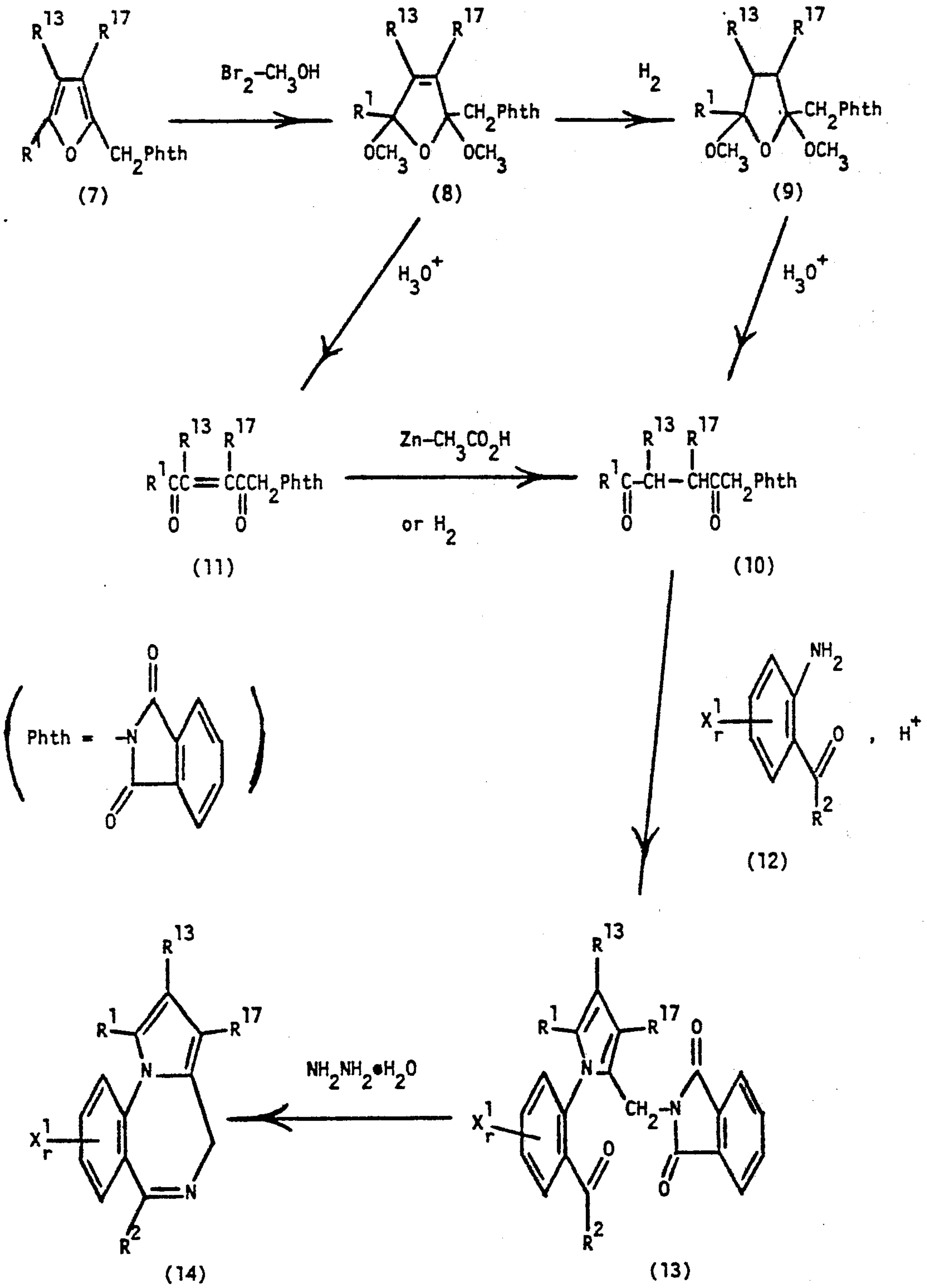


(7) (8) (9) (11) (10) (12) (13) (14)

SCHEME III

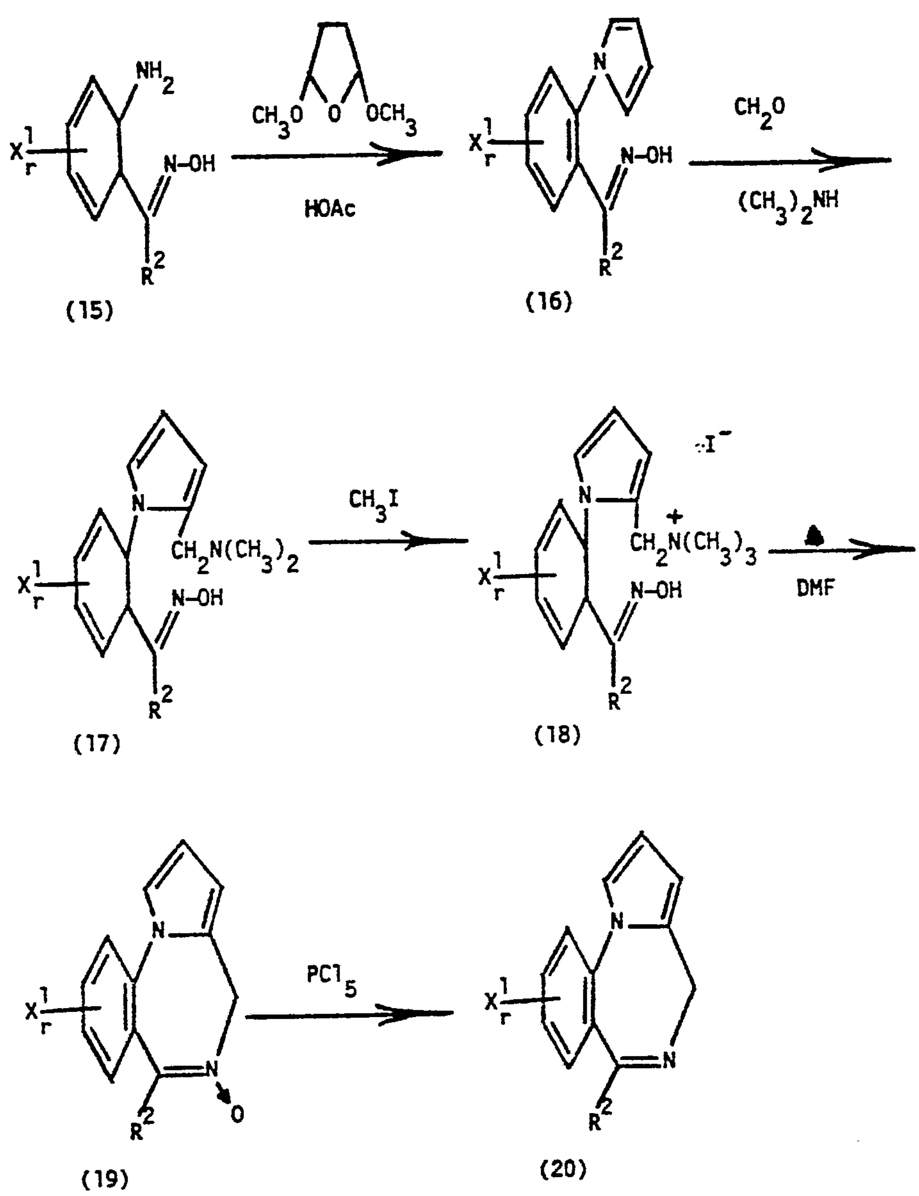

SCHEME IV

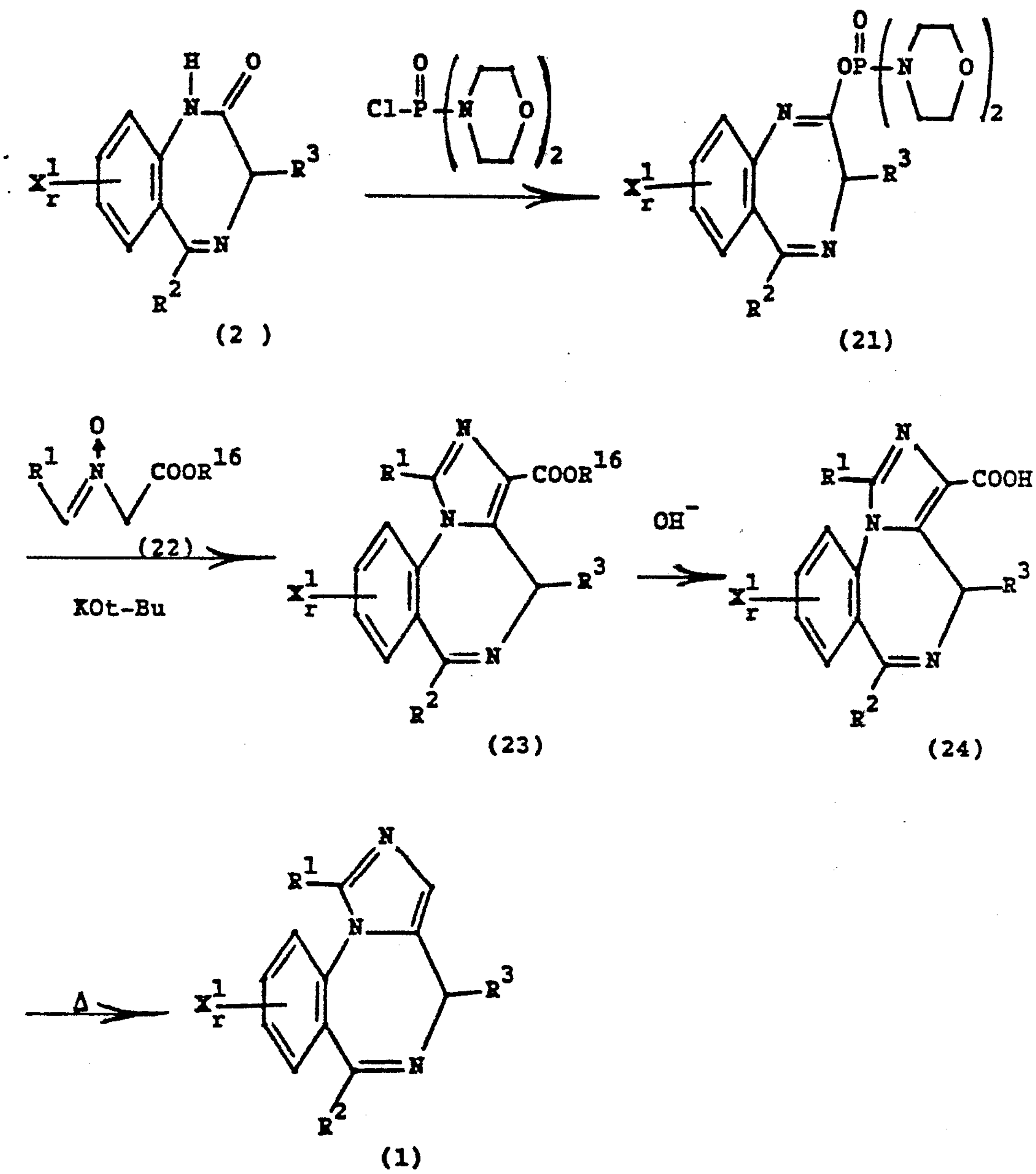


(2) (21) (23) (24) (1)

SCHEME V

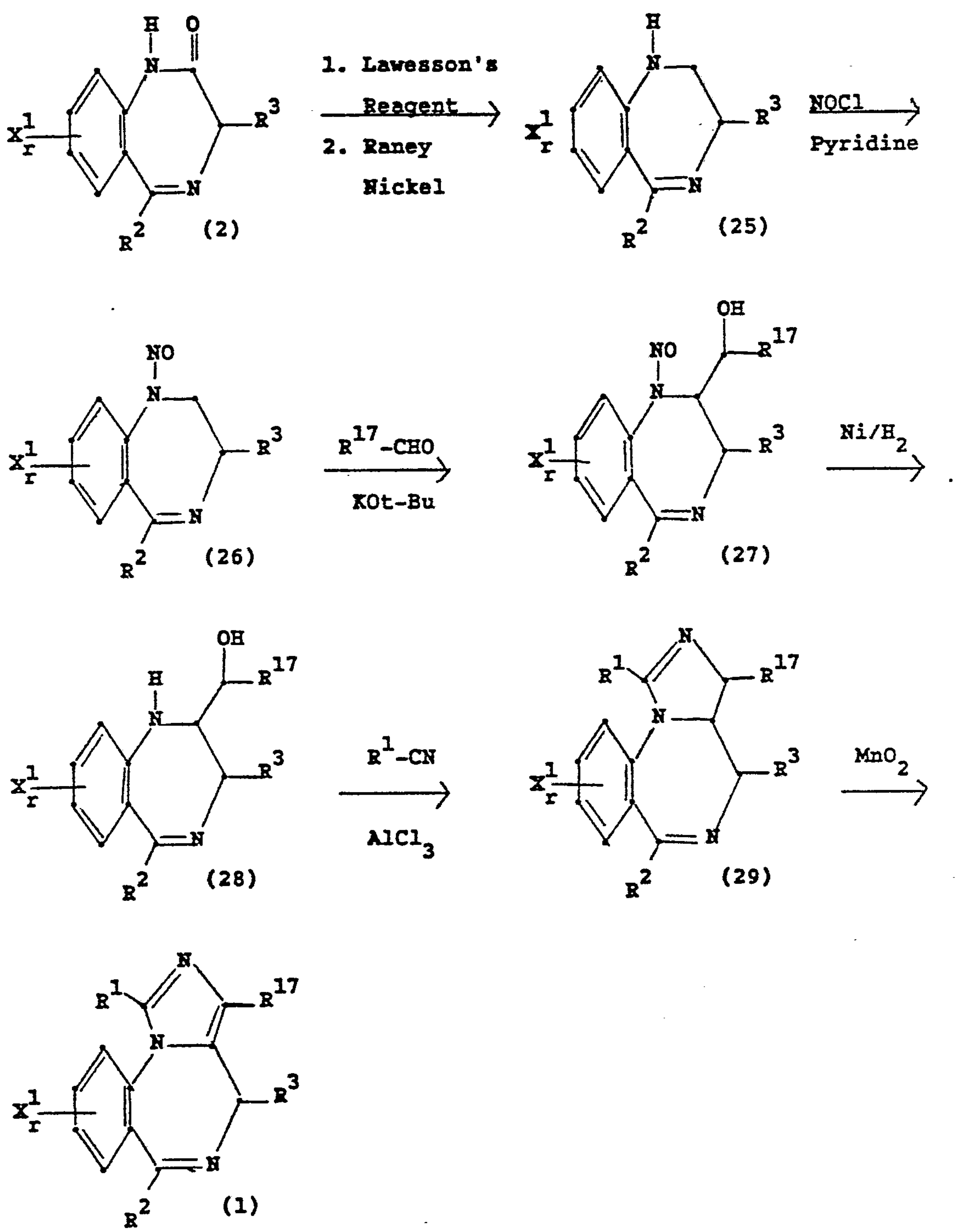


(2) (25) (26) (27) (28) (29) (1)

SCHEME VI

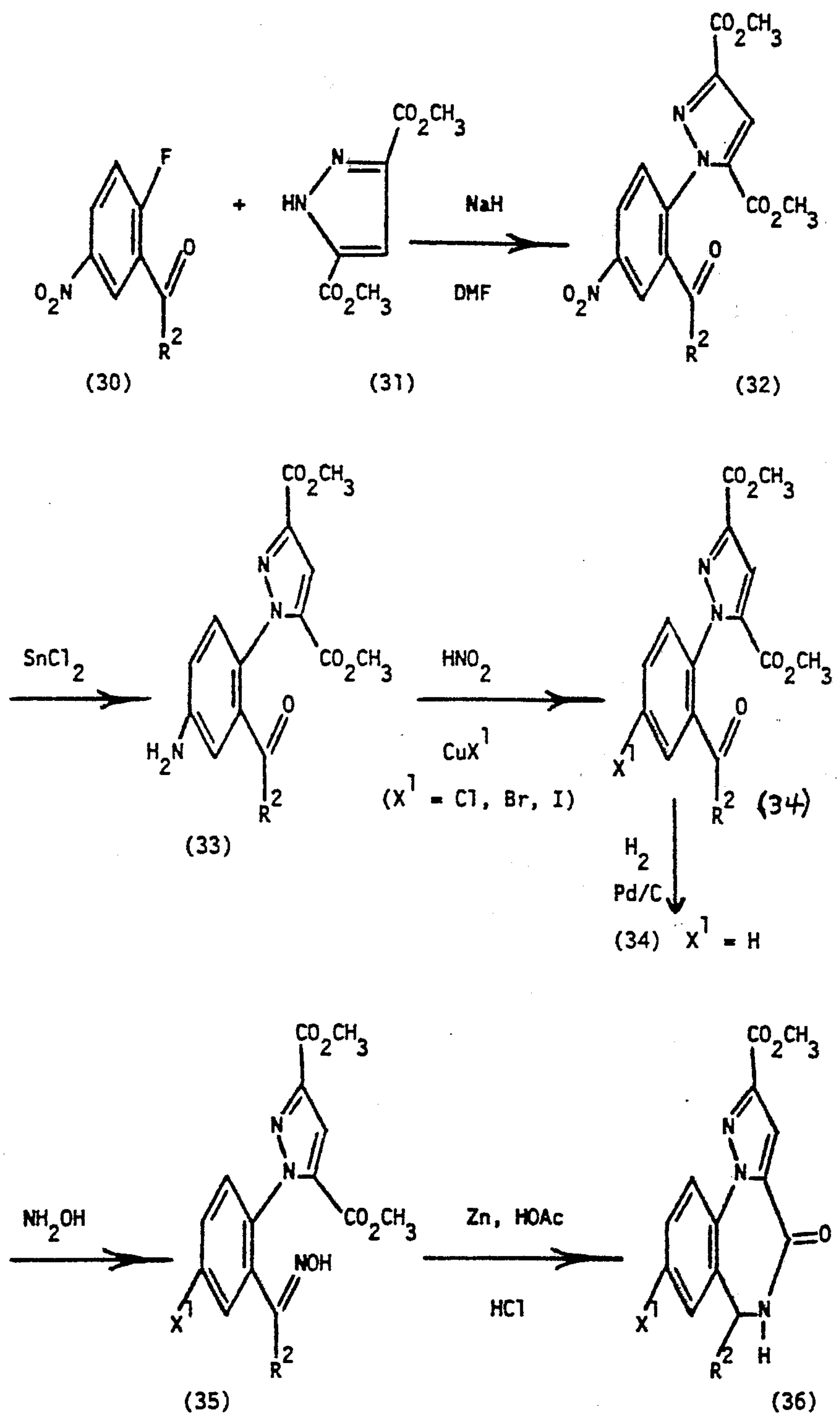
+
NaH
DMF
(30)
(31)
(32)
$SnCl_2$
$HNO_2$
$CuX^1$
($X^1$ = Cl, Br, I)
(33)
(34)
$H_2$
Pd/C
(34) $X^1$ = H
$NH_2OH$
Zn, HOAc
HCl
(35)
(36)

SCHEME VI (Cont.)

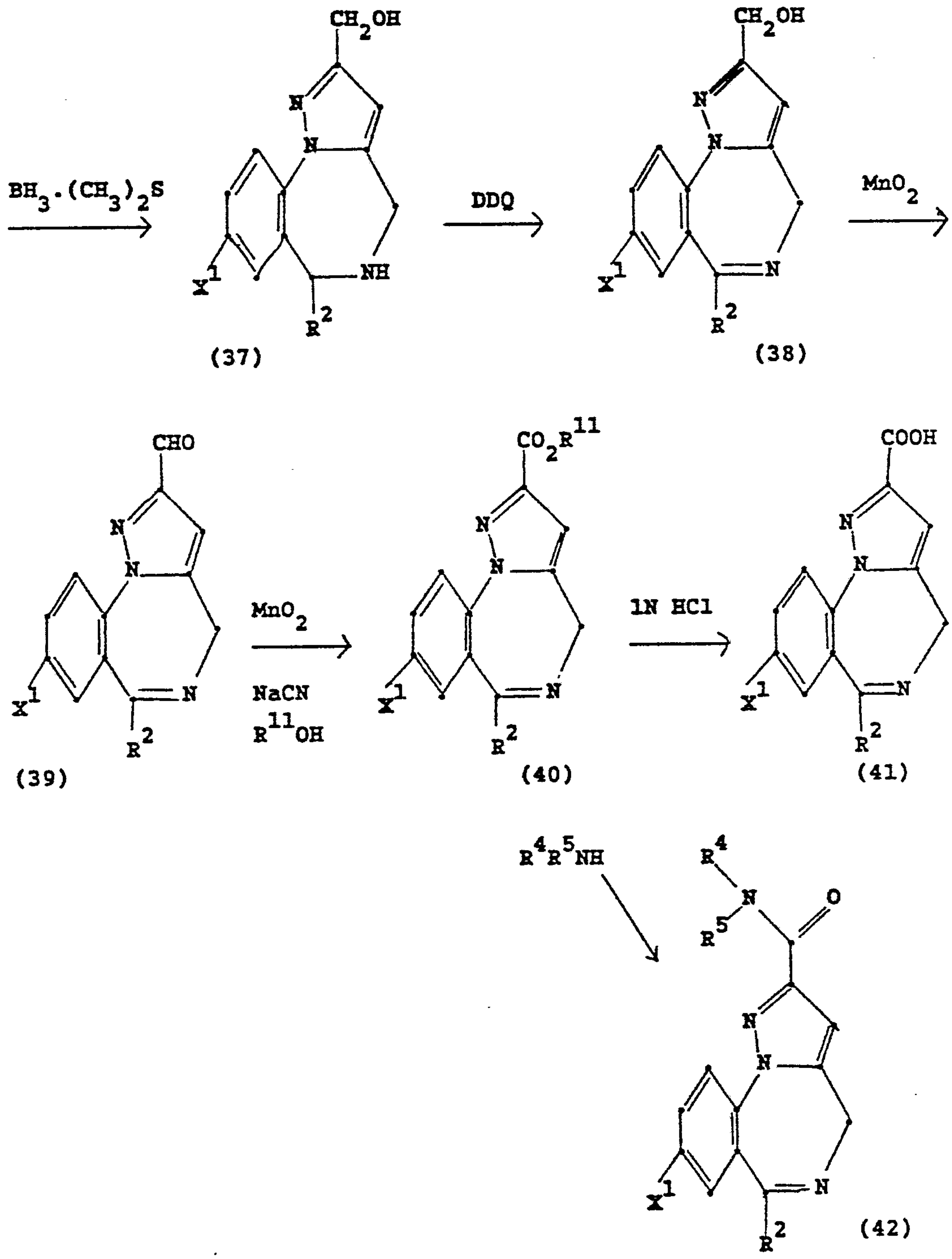


(37) (38) (39) (40) (41) (42)

SCHEME VI (Cont.)

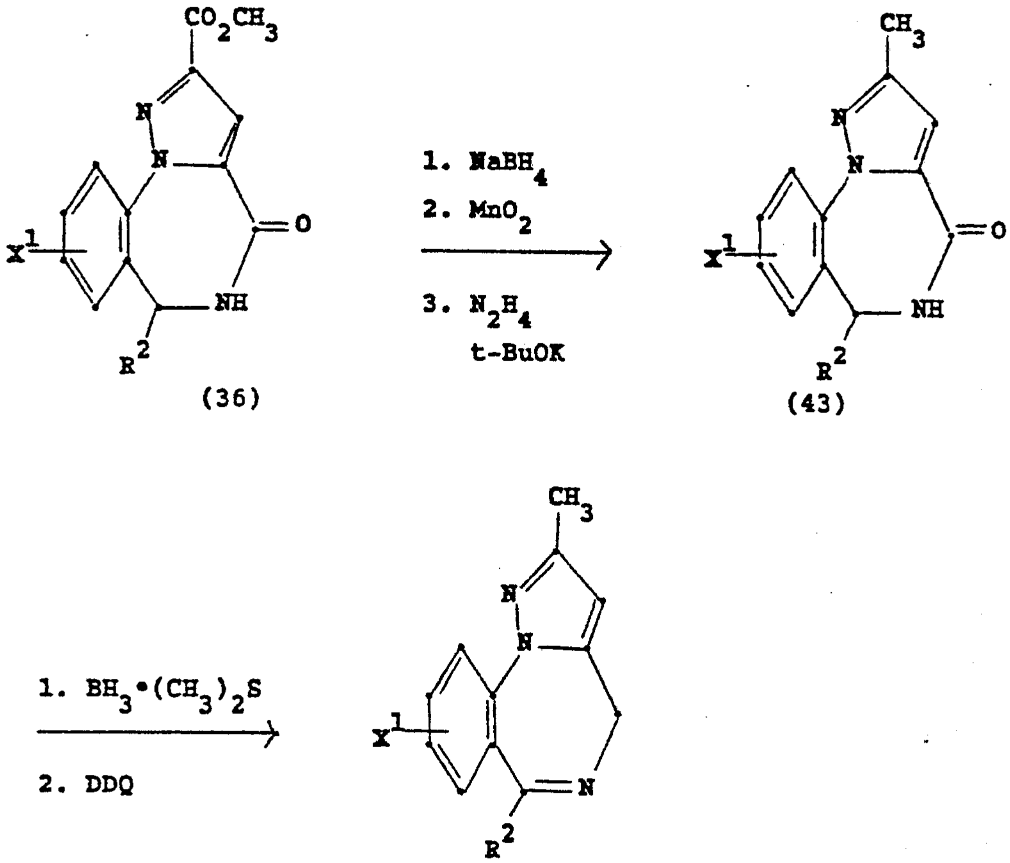


(36) (43) (44)

SCHEME VII

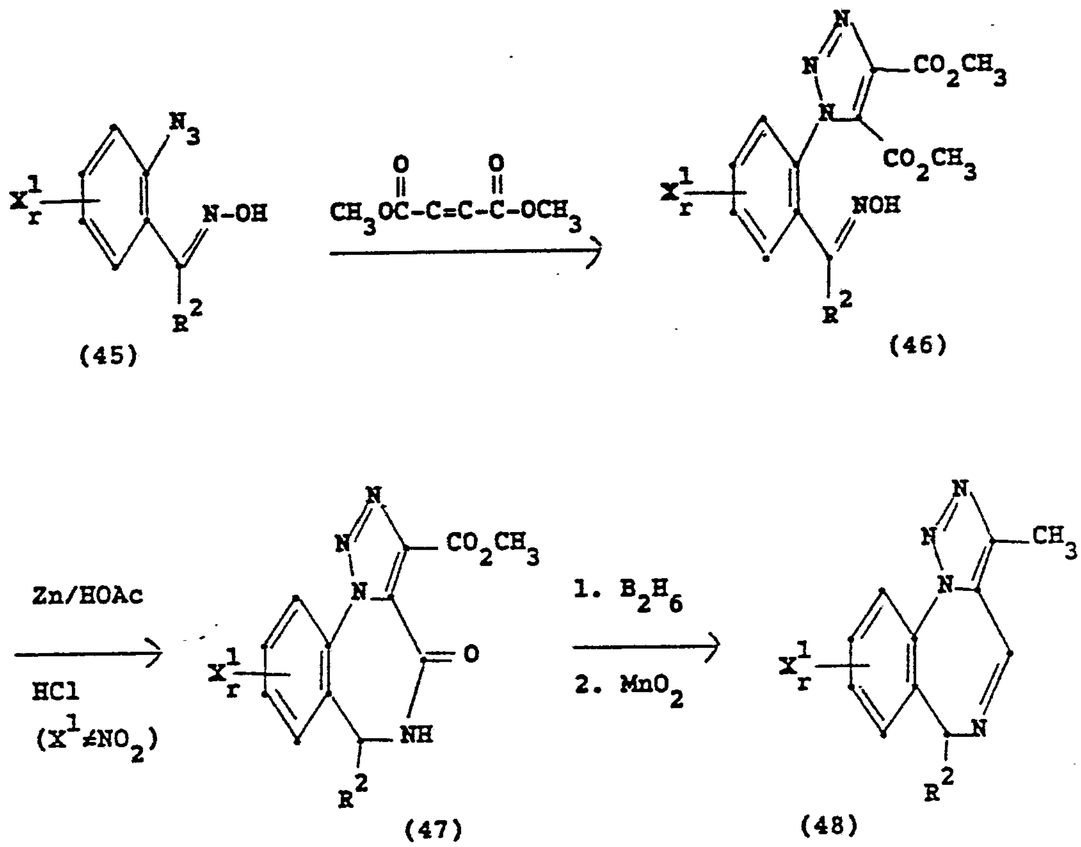


(45) (46) (47) (48)

SCHEME VIII

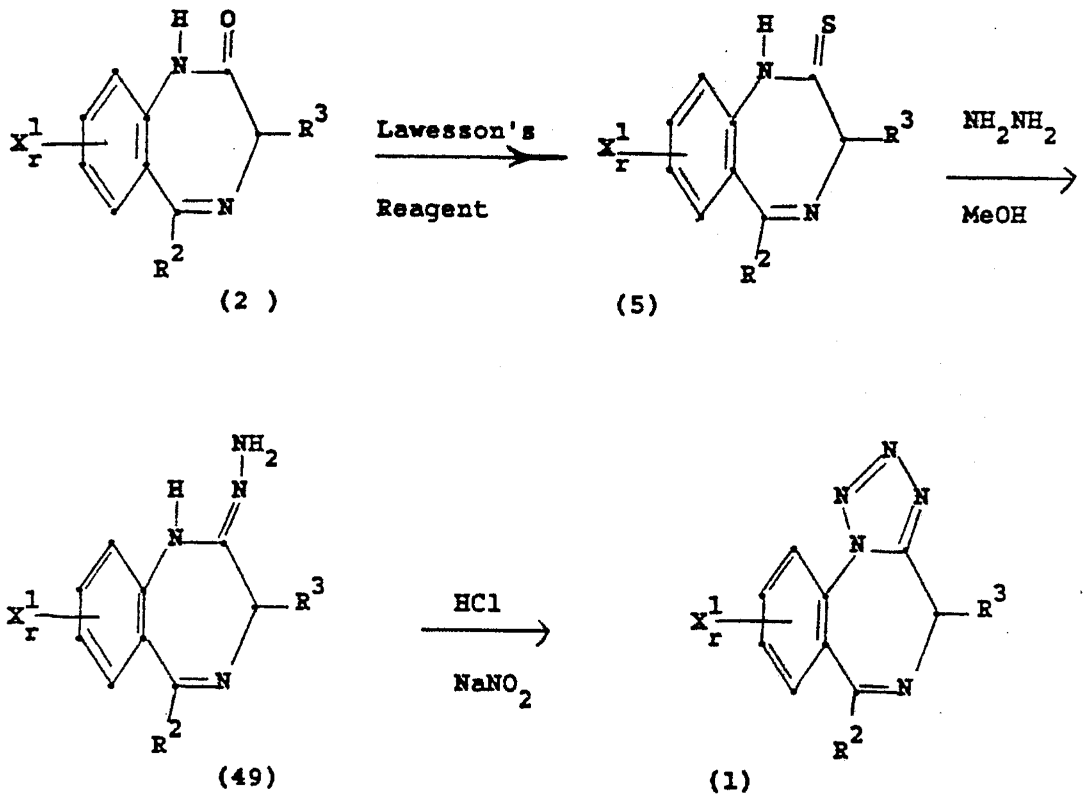


(2) (5) (49) (1)

REACTION SCHEME IX

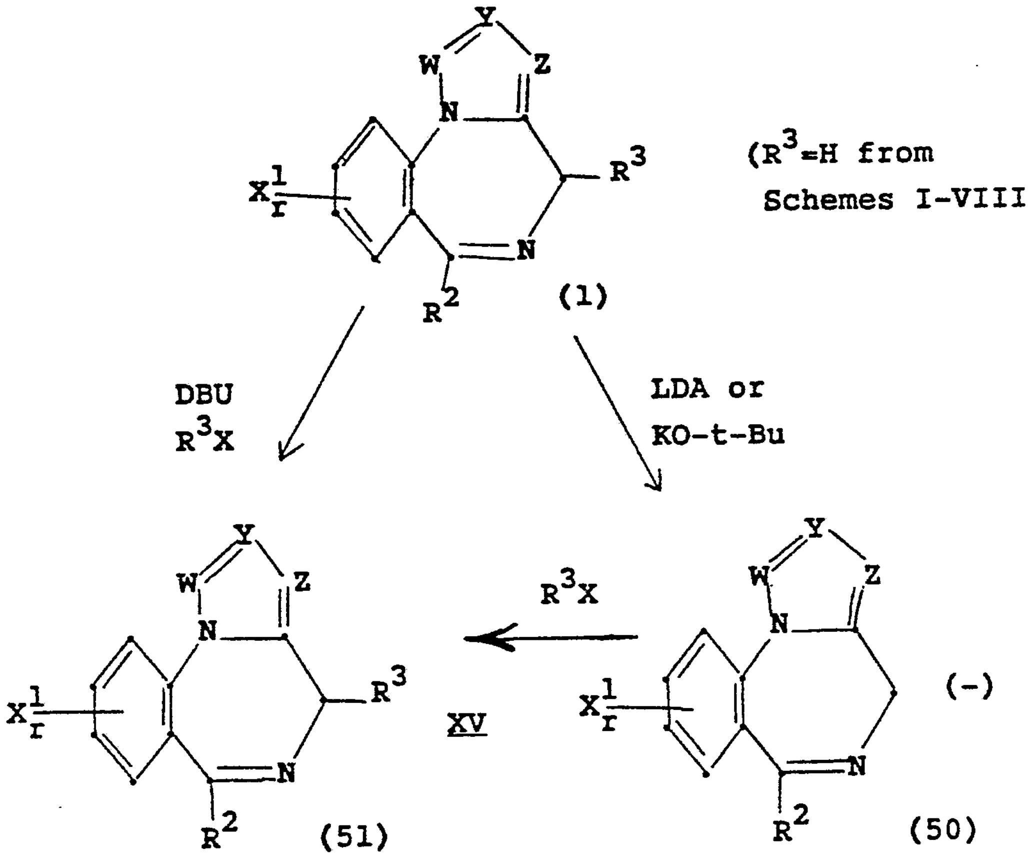


($R^3$=H from Schemes I-VIII)

(1)

XV

(51)

(–)

(50)

(n is at least 1 where the attachment atom to $R^7$ is C; otherwise n is at least 2)

REACTION SCHEME IX (cont'd)

(50)

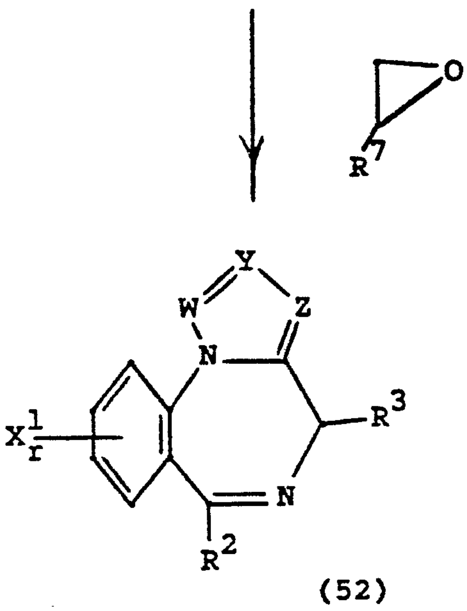

(52)

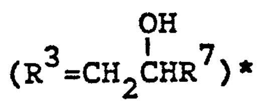

(50)

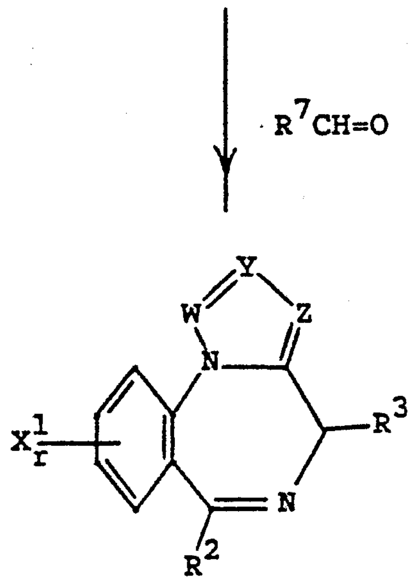


(53)

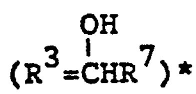

* (except where the attachment atom of $R^7$ is other than C)

REACTION SCHEME X

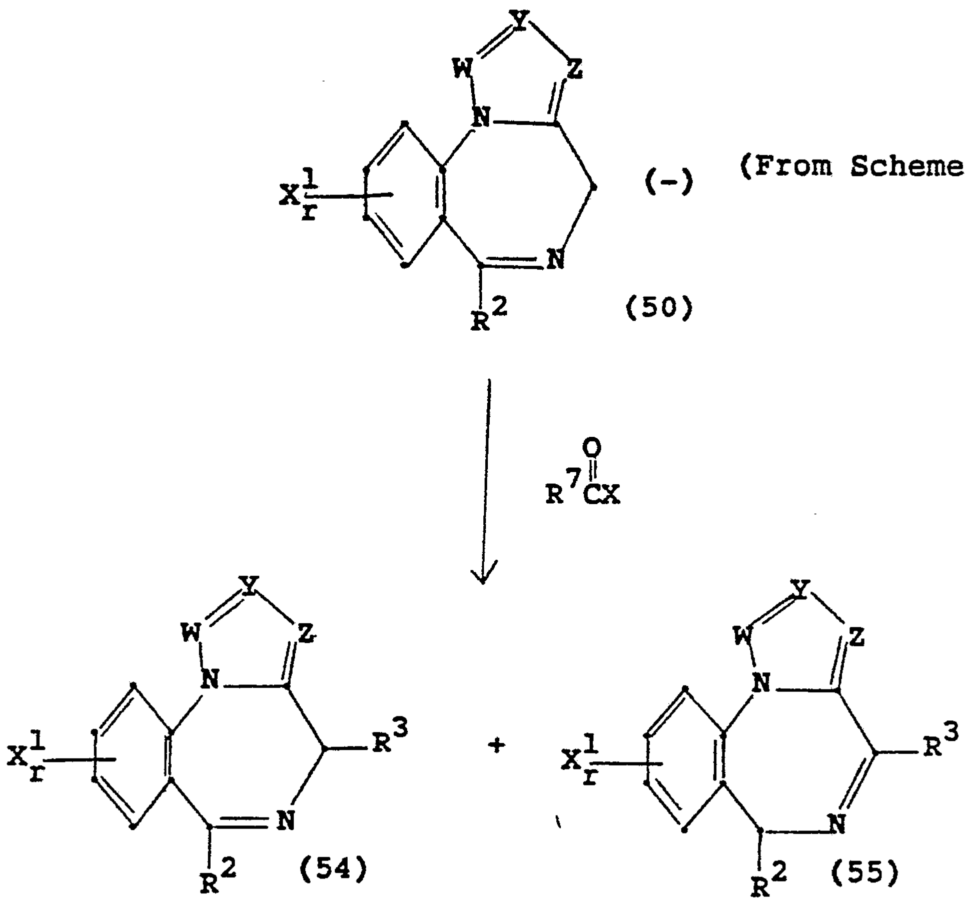

(-) (From Scheme IX)

(50)

(54) + (55)

$(R^3{=}\overset{\overset{O}{\|}}{C}{-}R^7)$

REACTION SCHEME X (Cont'd)

or (if peroxide present)

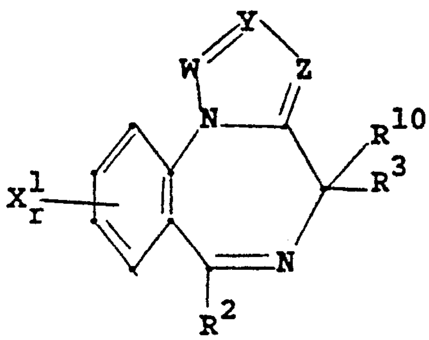

(56)

+

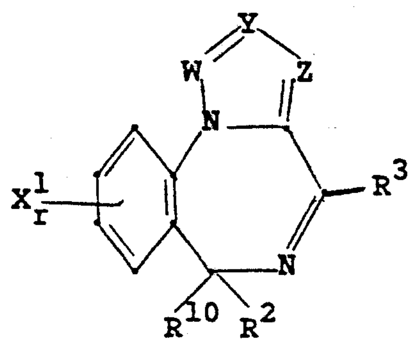

(57)

$(R^3 = \overset{O}{\overset{\|}{C}}R^7)$
$(R^{10} = OH)$

$(R^3 = \overset{O}{\overset{\|}{C}}R^7)$
$(R^{10} = OH)$

(except where atom adjacent to $R^7$ is other than C)

REACTION SCHEME XI

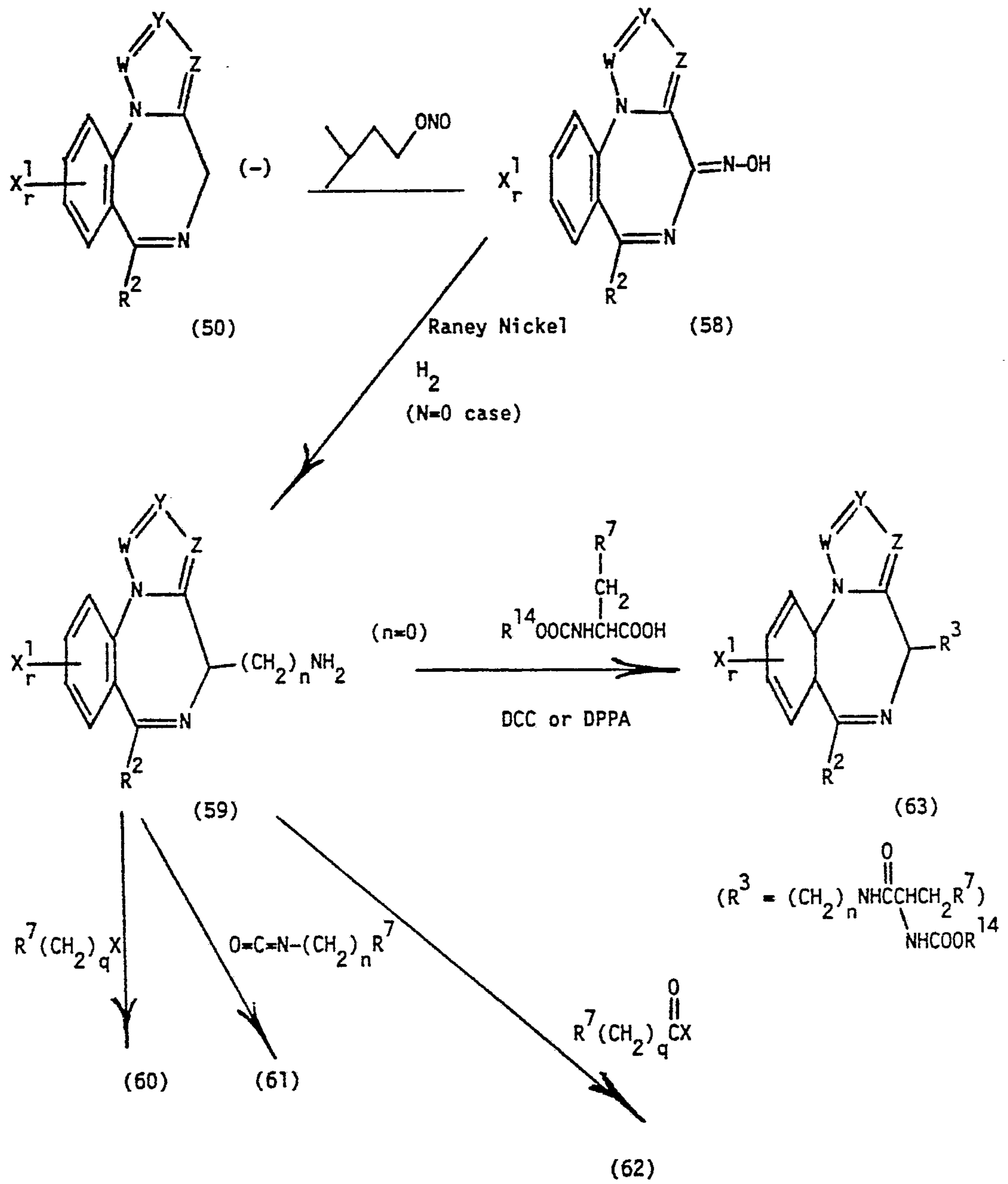


(50) (58) (59) (63) (60) (61) (62)

REACTION SCHEME XI (Cont'd)

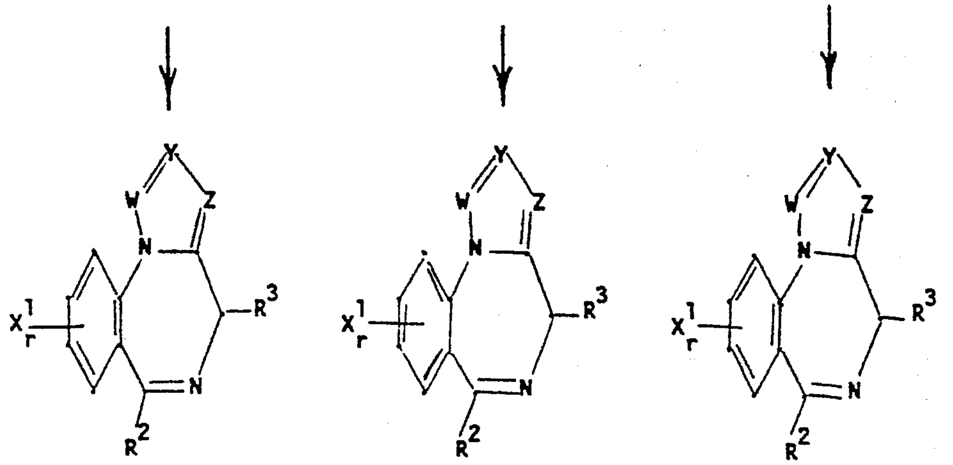

(60) (61) (62)

$R^3 = (CH_2)_nNH(CH_2)_qR^7$

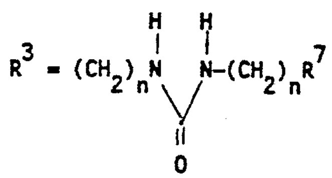

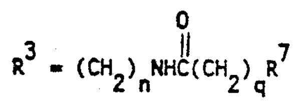

Referring to Reaction Scheme I, the benzodiazepines of Formula (2) which are readily available are used as the starting materials and are treated first with sodium methoxide and then with carbobenzoxybromoethylamine to give (3). Compound (3) is treated with a solution of HBr in glacial acetic acid to give the free amino derivative, and its dehydration product (4) formed by heating the amine under reflux in ethanol. Oxidation of (4) with manganese dioxide then produces the imidazobenzodiazepine of Formula (I). These latter compounds are also prepared by converting the compound of Formula (2) into its thioamide (5) with Lawesson's reagent. Treatment with primary amine derivatives and heating in ethanol gives (6), which upon exposure to concentrated sulfuric acid gives the imidazobenzodiazepines (1). Alternate syntheses of Formula (I) compounds use the compound of Formula (I) ($R^3$=H) and proceed according to Schemes IX, X, and XI.

Referring to Reaction Scheme II, treatment of 5-phthalimidomethylfuran (7) with bromine in a mixture of methylene chloride and absolute methanol at -40 to -50° C followed by introduction of gaseous ammonia gives the dihydrofuran (8) in an approximately equimolar mixture of trans and cis isomers.

Catalytic hydrogenation of (8) with the higher melting point using Raney nickel in ethanol affords the tetrahydrofuran (9). Hydrolysis of (9) gives ring-opened diketone (10). Alternatively, (10) may be prepared from (8) by hydrolysis to (11) followed by reduction with either zinc in acetic acid or by hydrogenation. Condensation of 2-aminobenzophenone (12) with (9) in benzene with p-toluenesulfonic acid as catalyst gives the pyrrolylketone (13).

Removal of the phthaloyl group from (13) and ring closure to 1-methyl-6-phenyl-4H-pyrrolo[1,2-a]-[1,4]benzodiazepine (14) is effected by heating at reflux a solution of (13) in ethanol with hydrazine hydrate. Most of the phthalazine generated in the reaction may be removed as solid by filtration from the reaction mixture. Chromatographic isolation followed by recrystallization gives pyrrolobenzodiazepine (14). Compound (14) may then be converted to compounds of Formula I according to Schemes IX-XI.

An alternate route to pyrrolobenzodiazepines is outlined in Scheme III. Using the method of Clauson-Kaas for the synthesis of 1-substituted pyrroles, aminobenzophenone oximes (15) are treated with 2,5-dimethoxytetrahydrofuran to give the corresponding 2-(1-pyrrolyl) ketone derivatives (16). Treatment of the oximes with formaldehyde and dimethylamine then gives the Mannich bases (17), which are alkylated with methyl iodide to afford the corresponding quaternary salts (18). Cyclization to N-oxides (19) is achieved by heating in DMF and deoxygenation with $PCl_5$ gives pyrrolobenzodiazepines (20).

Compound (20) is converted to pyrrolobenzodiazepine compounds of Formula I according to Schemes IX-XI.

Referring to Reaction Scheme IV, imidazobenzodiazepines of Formula I are prepared using benzodiazepines of Formula (2) as starting materials. The formula (2) compound is converted to iminophosphate (21) with dimorpholino phosphorochloridate. Condensation of the iminophosphate with the anion of the known nitrone, Compound 22, gives the imidazobenzodiazepine (23) in one step. Hydrolysis and decarboxylation gives (1) via (24).

A different approach is employed for the synthesis of the 1,3-substituted derivatives (1) (Scheme V). Compound (25) is synthesized from the Formula (2) benzodiazepine by conversion to the thioamide with Lawesson's reagent and desulfurization with Raney nickel. The 1-nitrosobenzodiazepine (26), which is readily obtained by reaction of compound (25) with nitrosyl chloride in pyridine, is condensed with aldehydes in the presence of potassium t-butoxide to give the diastereomeric alcohols (27). The diastereomers may be separated by chromatography if needed. Compounds (27) may then be reduced catalytically with Raney nickel to yield the corresponding amino alcohols (28).

Condensation of the amino alcohols (28) with acetonitrile and aluminum chloride gives the imidazoline (29) which need not be characterized, but is directly oxidized with activated manganese dioxide to yield the imidazole (1).

Certain compounds of Formula I are also prepared from imidazobenzodiazepines (1) ($R^3 = H$) according to the methods of Schemes IX-XI.

Referring to Reaction Scheme VI, the treatment of ketone (30) with the sodium salt of the pyrazolo diester, (31), in dimethylformamide leads to the ketone (32). The conversion of the nitro group to a halo substituent is carried out by a Sandmeyer procedure involving reduction of (32) with stannous chloride to give the amino compound (33). Diazotization with nitrous acid followed by treatment with cuprous halide gives the halo compound (34). Deshalo (34) is then obtained by hydrogenolysis with palladium on carbon. The synthesis of the pyrazolo[1,5-a][1,4]benzodiazepine ring system is achieved by the oximation of (34) to compound (35) which is reductively cyclized to (36) by treatment with zinc in acetic acid.

The reduction of the lactam ester, (36), to the dihydrobenzodiazepine, (37), is carried out with excess boranedimethylsulfide complex which reduces both the lactam and ester groups. The use of diborane in tetrahydrofuran gives a mixture of (37) in addition to the partially-reduced amino-ester. The oxidation of (37) with 2,3-dichloro-5,6-dicyano-1,4-benzoquinone (DDQ) yielded 8-halo[1,5-a][1,4]-benzodiazepine (38).

The alcohol (38) is oxidized to the aldehyde (39) with manganese dioxide, rather than oxidizing the dihydroalcohol (37) directly to (39), which would result in lower overall yields.

The aldehyde, (39), is a versatile intermediate for the preparation of various 2-substituted pyrazolobenzodiazepines. The carboxylic ester, (40), is prepared from (39) by treatment with manganese dioxide in an alcohol in the presence of sodium cyanide. The ester is subsequently converted either to the acid, (41), by acid hydrolysis or to the amides, (42) by treatment with amines such as ammonia, methylamine and dimethylamine.

An additional pyrazolobenzodiazepine, the 2-methyl analog, is prepared starting with compound (36). The ester function is reduced with sodium borohydride to the alcohol, with oxidation with manganese dioxide giving the aldehyde, which in turn, under Wolff-Kishner conditions, gives the methyl compound (43). The product (44) is obtained by reduction of the amide group of (43) followed by DDQ oxidation.

Compound (44) is converted to the compounds of Formula I according to the methods of Schemes IX-XI.

Referring to Reaction Scheme VII, azido compound (45) undergoes a 1,3-dipolar cycloaddition with dimethylacetylene dicarboxylate to yield compound (46). The oxime of (46) is reduced with zinc in acetic acid which results in cyclization to amide (47). Reduction of the amide with diborane followed by oxidation with manganese dioxide produces (48) in which the ester is reduced to methyl. Compounds (48) are converted to Formula I compounds according to Schemes IX-XI.

Referring to Reaction Scheme VIII, thioamides (5) are converted to amidrazones (49) with hydrazine in methanol. Diazotization then produces tetrazolobenzodiazepines (1), with compounds (1) ($R^3 = H$) being converted to other compounds of Formula I according to Scheme IX-XI.

Referring now to Reaction Scheme IX, the anion (50) is generated from (1) by the procedure of J. Org. Chem., 46, 3945 (1981) using lithium diisopropylamide (LDA) or using potassium tert-butoxide.

The hydroxy alkyl derivative (53) is generated by adding an aldehyde to a solution of (50). Treatment of (50) with an epoxide yields the hydroxyethyl derivative (52) and by treating (50) with an alkyl halide, the alkyl derivative (51) is produced.

An alternative procedure for obtaining (51) is to treat (1) with an alkyl halide and a strong base such as 1,8-diazabicyclo[5.4.0]undec-7-ene (DBU) and heating.

These procedures also produce isomers of (51)-(53) which are analogous to (55) (Reaction Scheme X). Likewise, in the presence of peroxide, the analogs of the isomers and hydroxy derivatives (56) and (57) are produced.

Reaction Scheme X describes the formation of $R^3 =$ keto compounds of Formula I. These are produced by treating the anion (50) with an acid halide or anhydride, with this reaction producing both isomers (54) and (55). When the reaction is run in the presence of peroxide, the hydroxy compounds (56) and (57) are produced.

Reaction Scheme XI describes the formation of Formula I compounds where $R^3$ is a substituted amino or aminomethyl.

The heterocycle-fused benzodiazepines (59) are either known or readily derivable from known compounds. The amino compounds may also be obtained by nitrosation of (50) followed by reduction of the oxime (58) with Raney nickel and hydrogen.

When (59) is treated with an alkyl halide, the N-alkyl derivative (60) is produced.

When (59) is treated with an alpha-halo carboxylic acid derivative such as an $\alpha$-halo acid, ester, amide, or the like, one obtains the corresponding $\alpha$-amino compound (61).

Treatment of (59) with an acid halide or anhydride produces the N-acyl derivative (62).

Compound (59) may also be treated with an N-protected α-amino acid and a coupling reagent such as DCC or DPPA (diphenylphosphorylazide) to give the amides of structure (63).

Treatment of Compound (59) with an isocyanate gives the ureas (61).

The pharmaceutically-acceptable salts of the present invention may be synthesized from the compounds of Formula I which contain a basic moiety by conventional chemical methods. Generally, the salts are prepared by reacting the free base with stoichiometric amounts of or with an excess of the desired salt-forming inorganic or organic acid in a suitable solvent or in various combinations of solvents.

Screening of the novel compounds according to the present invention to determine biological activity and obtain an $IC_{50}$ value for them, in order to identify significant CCK-antagonism, may be accomplished using an $^{125}$I-CCK-receptor binding assay and in vitro isolated preparations. In order to identify significant gastrin antagonism, $^{125}$I-gastrin and $^{3}$H-pentagastrin binding assays may be used. These tests involve the following:

CCK receptor binding (pancreas) method

CCK-8, radiolabeled with $^{125}$I-Bolton Hunter reagent (2000 Ci/mmole) is purchased from New England Nuclear (NEN) and receptor binding is performed according to Innis and Snyder (Proc. Natl. Acad. Sci., 77, 6917-6921, 1980), with minor modifications as described in Chang and Lotti (Proc. Natl. Acad. Sci., 83, 4923-4926, 1986).

The whole pancreas of a male Sprague-Dawley rat (200-350 g), which has been sacrificed by decapitation is dissected free of fat tissue and homogenized in 20 volumes of ice-cold 50 mM Tris HCl (pH 7.7 at 25° C) with a Brinkmann Polytron PT-10. The homogenates are centrifuged at 48,000 g for 10 minutes, then the resulting pellets are resuspended in Tris Buffer, centrifuged as above, and resuspended in 200 volumes of binding assay buffer (50 mM Tris HCl, pH 7.7 at 25° C, 5 mM dithiothreitol and 0.1 mM bacitracin).

For the binding assay, 25 μl of buffer (for total binding), or unlabeled CCK-8 sulfate sufficient to give a final concentration of 1 μM of CCK-8 (for nonspecific binding), or the compounds according to the instant invention (for determination of antagonism to $^{125}$I-CCK binding) and 25 μl of $^{125}$I-CCK-8 (30,000-40,000 cpm), are added to 450 μl of the membrane suspensions in duplicate or triplicate test tubes. The reaction mixtures are incubated at 37° C for 30 minutes and then filtered on glass fiber GF/B filters, which are then rapidly washed with 3 × 4 ml of ice cold Tris HCl containing 1 mg/ml BSA, and the filters are counted with a Beckman Gamma 5000. For Scatchard analysis to determine the mechanism of inhibition of $^{125}$I-CCK binding by the most potent compounds (Ann. N.Y. Acad. Sci., 51, 660, 1949), $^{125}$I-CCK-8 is progressively diluted with increasing concentrations of CCK-8.

CCK receptor binding (brain) method

$^{125}$I-CCK-8 binding is performed similarily to the method described by Saito et al. (J. Neurochem., 37 483-490, 1981), with modifications described by Chang and Lotti (Proc. Natl. Acad. Sci., 83, 4923-4926, 1986).

Male Hartley guinea pigs (300-500 g) are sacrificed by decapitation, and the brains are removed and placed in ice-cold 50 mM Tris HCl (Trizma-7.4) [pH 7.4 at 25°C]. The cerebral cortex is dissected and used as a receptor source and each gram of fresh guinea pig brain tissue is homogenized in 10 ml of Tris/Trizma buffer with a Brinkmann polytron PT-10. The homogenates are centrifuged at 42,000g for 15 minutes, then the resulting pellets are resuspended in 200 volumes of binding assay buffer (10 mM N-2-hydroxyethylpiperazine-N′-2-ethanesulfonic acid (HEPES), 5 mM $MgCl_2$, 1 mM ethylene glycol-bis-(β-aminoethylether)-N,N′-tetraacetic acid (EGTA), 0.4% BSA (bovine serum albumin) and 0.25 mg/ml bacitracin, (pH 6.5).

The remainder of the binding assay method is as described for the pancreas method, except that the reaction mixtures are incubated at 25° C for 2 hours before centrifugation.

Isolated guinea pig gall bladder method

The two halves of the gall bladders, free of adjacent tissue, of male Hartley guinea pigs (400-600g), which have been sacrificed by decapitation, are suspended under 1g tension along the axis of the bile duct in 5 ml organ bath, containing a Kreb's bicarbonate solution of 118 mM NaCl, 4.75 mM KCl, 2.54 mM $CaCl_2$, 1.19 mM $KH_2PO_4$, 1.2 mM $MgSO_4$, 25 mM $NaHCO_3$ and 11 mM dextrose, which is maintained at 32° C and bubbled with a mixture of 95% $O_2$ and 5% $CO_2$. The tissues are washed every 10 minutes for one hour to obtain equilibrium prior to the beginning of the study and the isometric contractions of the strips are recorded using Statham (60g:0.12 mm) strain gauges and a Hewlett-Packard 77588 recorder.

CCK-8 is added cumulatively to the baths and $EC_{50}$'s are determined using regression analysis. After washout (every 10 minutes for one hour), the compound to be tested is added at least 5 minutes before the addition of CCK-8 and the $EC_{50}$ of CCK-8 in the presence of compound to be tested is similarly determined.

A shift to the right of the CCK dose response curve without reduction of the maximal centractile response, indicates competitive antagonism of CCK from this method.

Isolated longitudinal muscle of guinea pig ileum

Longitudinal muscle strips with attached nerve plexus are prepared as described in Brit. J. Pharmac. 23: 356-363, 1964; J. Physiol. 194: 13-33, 1969. Male Hartley guinea pigs are decapitated and the ileum removed (10 cm of the terminal ileum is discarded and the adjacent 20 cm piece used), with a 10 cm piece of the ileum being stretched on a glass pipette. Using a cotton applicator to stroke tangentially away from the mesentery attachment at one end, the longitudinal muscle is separated from the underlying circular muscle and the

longitudinal muscle is tied to a thread and by gently pulling, stripped away from the entire muscle. A piece of approximately 2 cm is suspended in 5 ml organ bath containing Krebs solution and bubbled with 95% $O_2$ and 5% $CO_2$ at 37°C under 0.5 g tension. CCK-8 is added cumulatively to the baths and $EC_{50}$ values in the presence and absence of compounds to be tested are determined, as described in the gall bladder protocol above.

Gastrin Receptor Binding in Guinea Pig Gastric Glands

Guinea pig gastric mucosal glands are prepared by the procedure of Berglingh and Obrink, Acta Physiol. Scand. 96: (1976), with a slight modification according to Praissman et al. C. J. Receptor Res. 3: (1983). Gastric mucosa from male Hartley guinea pigs ( 300-500 g body weight) are washed thoroughly and minced with fine scissors in standard buffer consisting of the following: 130 mM NaCl, 12 mM $NaHCO_3$, 3 mM $NaH_2PO_4$, 3 mM $Na_2HPO_4$, 3 mM $K_2HPO_4$, 2 mM $MgSO_4$, 1 mM $CaCl_2$, 5 mM glucose, 4 mM L-glutamine and 25 mM HEPES at pH 7.4. The minced tissues are washed and incubated in a 37°C shaker bath for 40 minutes, with the buffer containing 0.1% collagenase and 0.1% BSA, and bubbled with 95% $O_2$ and 5% $CO_2$. The tissues are passed twice through a 5 ml glass syringe to liberate the gastric glands, and then filtered through 200 mesh nylon. The filtered glands are centrifuged at 270 g for 5 minutes and washed twice by resuspension and centrifugation.

The washed guinea pig gastric glands are resuspended in 25 ml of standard buffer containing 0.25 mg/ml of bacitracin. For binding studies, 10 µl of buffer (for total binding) or gastrin (1 µM final concentration, for nonspecific binding) or test compound and 10 µl of $^{125}$I-gastrin (NEN, 2200 Ci/mmole, 25 pM final) or $^{3}$H-pentagastrin (NEN, 22 Ci/mmole, 1 nM final) are added to 220 µl of gastric glands in triplicate tubes which are aerated with 95% $O_2$ and 5% $CO_2$, and capped. The reaction mixtures, after incubation at 25°C for 30 minutes, are filtered under reduced pressure on glass G/F B filters (Whatman) and immediately washed with 4 × 4 ml of standard buffer containing 0.1% BSA. The radioactivity on the filters is measured using a Beckman gamma 5500 for $^{125}$I-gastrin or liquid scintillation counting for $^{3}$H-pentagastrin.

The ability of the instant 3-substituted 1,4-benzodiazepines with 5-membered heterocyclic rings to antagonize CCK and gastrin makes these compounds useful as pharmaceutical agents for mammals, especially for humans, for the treatment and prevention of disorders wherein CCK and/or gastrin may be involved. Examples of such disease states include gastrointestinal disorders, especially such as irritable bowel syndrome or ulcers, excess pancreatic or gastric secretion, acute pancreatis, motility disorders or gastrointestinal neoplasms; central nervous system disorders, caused by CCK interactions with dopamine, such as neuroleptic disorders, tardive dyskinesia, Parkinson's disease, psychosis or Gilles de la Tourette Syndrome; disorders of appetite regulatory systems; Zollinger-Ellison syndrome, antral G cell hyperplasia, or pain (by the potentiation and prolongation of opiate-mediated analgesia); as well as certain tumors of the lower esophagus, stomach, intestines and colon.

The compounds of the instant invention or pharmaceutically-acceptable salts thereof, may be administered to a human subject either alone or, preferably, in combination with pharmaceutically-acceptable carriers or diluents, optionally with known adjuvants, such as alum, in a pharmaceutical composition, according to standard pharmaceutical practice. The compounds can be administered orally or parenterally, including intravenous, intramuscular, intraperitoneal, subcutaneous and topical administration.

For oral use of an antagonist of CCK, according to this invention, the selected compounds may be administered, for example, in the form of tablets or capsules, or as an aqueous solution or suspension. In the case of tablets for oral use, carriers which are commonly used include lactose and corn starch, and lubricating agents, such as magnesium stearate, are commonly added. For oral administration in capsule form, useful diluents include lactose and dried corn starch. When aqueous suspensions are required for oral use, the active ingredient is combined with emulsifying and suspending agents. If desired, certain sweetening and/or flavoring agents may be added. For intramuscular, intraperitoneal, subcutaneous and intravenous use, sterile solutions of the active ingredient are usually prepared, and the pH of the solutions should be suitably adjusted and buffered. For intravenous use, the total concentration of solutes should be controlled in order to render the preparation isotonic.

When a compound according to the instant invention, or a salt thereof, is used as an antagonist of CCK or gastrin in a human subject, the daily dosage will normally be determined by the prescribing physician with the dosage generally varying according to the age, weight, and response of the individual patient, as well as the severity of the patient's symptoms. However, in most instances, an effective daily dosage will be in the range of from about 0.05 mg/kg to about 50 mg/kg of body weight, and preferably, of from 0.1 mg/kg to about 20 mg/kg of body weight, administered in single or divided doses. In some cases, however, it may be necessary to use dosages outside these limits.

In the treatment of irritable bowel syndrome, for instance, 1 to 10 mg/kg of a CCK antagonist might be administered orally (p.o.), divided into two doses per day (b.i.d.). In treating delayed gastric emptying, the dosage range would probably be the same, although the drug might be administered either intravenously (I.V.) or orally, with the I.V. dose probably tending to be slightly lower due to better availability. Acute pancreatitis might be treated preferentially in an I.V. form, whereas spasm and/or reflex esophageal, chronic pancreatitis, past vagatomy diarrhea, or treatment of anorexia or of pain associated with biliary dyskinesia might indicate p.o. form administration.

In the use of a gastrin antagonist as a tumor palliative for gastrointestinal neoplasms with gastrin receptors,

as a modulator of central nervous system activity treatment of Zollinger-Ellison syndrome, or in the treatment of peptic ulcer disease, a dosage of .1 to 10 mg/kg administered one-to-four times daily might be indicated.

Because these compounds antagonize the function of CCK in animals, they may also be used as feed additives, in amounts of from about 0.05 mg/kg to about mg/kg of body weight, to increase the food intake of animals.

The invention is further defined by reference to the following examples which are intended to be illustrative and not limiting.

EXAMPLE 1

Preparation of 6-phenyl-4H-pyrrolo[1,2-a][1,4]benzodiazepine (14, $X^1 = H$, $R^2 = Ph$, $R^1 = R^{13} = R^{17} = H$)

This compound is prepared according to the method of Hara et al, J. Med. Chem., 21, 263-268 (1978).

EXAMPLE 2

Preparation of 4-oximino-6-phenyl-4H-pyrrolo [1,2-a]-1,4-benzodiazepine (58, $X^1 = H$, $R^2 = Ph$, $W = Y = Z = CH$)

To a suspension of potassium tert-butoxide (24.9 g, 222 mmole) in 600 ml of dry tetrahydrofuran is added 200 ml of dry tert-butylalcohol at -20°C under nitrogen. To this solution is then added, via addition funnel, 6-phenyl-4H-pyrrolo[1,2-a]-1,4-benzodiazepine (25 g) in 260 mL of tetrahydrofuran. The resulting solution is stirred for about 2 hours at -20° C and treated with 17.4 mL (130 mmole) of isoamyl nitrite. The reaction mixture is warmed to 0° C over approximately 15 minutes and quenched with the addition of 60 mL of cold water and 20 mL of glacial acetic acid. All solvents are removed under reduced pressure and the residue is partitioned between ethyl acetate (600 mL) and brine (100 mL). The phases are separated and the organic extracts are dried ($Na_2SO_4$) and concentrated. The resulting product is triturated with ether to give Compound (58).

EXAMPLE 3

Preparation of 4(R,S)-amino-6-phenyl-4H-pyrrolo [1,2-a]-1,4-benzodiazepine (59, $X^1 = H$, $R^2 = Ph$, $W = Y = Z = CH$, $n = O$)

A solution of 150 mL of methanol containing 5 g 4-oximino-6-phenyl-4H-pyrrolo[1,2-a]-1,4-benzodiazepine is treated with a slurry of active Raney-nickel catalyst[1] in ethanol (10 g). The resulting suspension is hydrogenated on a Parr apparatus at 60 psi and 23° C for about 30 hour. The catalyst is removed by filtration and the filtrate is concentrated to afford the title compound.

EXAMPLE 4

Preparation of 4(R,S)-(2(S)-tert-butoxycarbonylamino-3-phenylpropanoylamino)-6-phenyl-4H-pyrrolo[1,2-a]-1,4-benzodiazepine

Crude 4(R,S)-amino-6-phenyl-4H-pyrrolo-[1,2-a]-1,4-benzodiazepine (1.37 g), Boc-L-phenylalanine (1.37 g, 5.17 mmole), 1-hydroxybenzotriazole (HBT) (0.70 g, 5.17 mmole), and 1-ethyl-3-(3-dimethylaminopropyl)carbodiimide hydrochloride (EDC) (0.99 g, 5.17 mmole) are combined in DMF (30 mL) and stirred at room temperature. The pH of the reaction mixture is adjusted to 8.5 with triethylamine. After approximately 1/2 hour, the DMF is removed in vacuo and the residue is partitioned between ethyl acetate and 10% citric acid solution (10 mL). The layers are separated and the organic phase is washed with sodium bicarbonate solution ($NaHCO_3$, saturated). The combined organic layers are washed with brine, dried over $Na_2SO_4$, filtered, and evaporated to dryness in vacuo. The residue is chromatographed on silica gel and the combined product fractions evaporated to dryness in vacuo to give the title compound as a mixture of diastereomers.

EXAMPLE 5

Preparation of 4(R and S)-(2(S)-Amino-3-phenylpropanoylamino-6-phenyl-4H-pyrrolo[1,2-a]-1,4-benzodiazepine

4(RS)-(2(S)-tert-Butoxycarbonylamino-3-phenylpropanoylamino)-6-phenyl-4H-pyrrolo[1,2-a]-1,4-benzodiazepine (1.8 gm) is dissolved in EtOAc (25 mL), cooled to 0° C, and the solution saturated with HCl (g) over a 10 minute period. After stirring an additional 10 minutes, the solvent is removed in vacuo. The residue is dissolved in $H_2O$, basified with saturated $Na_2CO_3$ (aqueous) and extracted with EtOAc (3×). The organic layers are combined, washed with brine, dried over $Na_2SO_4$, filtered and rotoevaporated in vacuo to give a foam. Flash chromatography on silica gel separates the 1/1 pair of diastereomers into an upper and a lower component. The individual fractions containing the components are concentrated to dryness to give the separated diastereomers.

[1] The Raney-Nickel catalyst is prepared according to Fieser & Fieser, Reagents for Organic Synthesis, Vol. I, John Wiley & Sons, Inc., New York 1967, p. 729.

## EXAMPLE 6

Preparation of 4(R)- and 4(S)-Amino-6-phenyl-4H-pyrrolo[1,2-a]-1,4-benzodiazepine

4(S)-(2(S)-amino-3-phenylpropanoylamino)-6-phenyl-4H-pyrrolo[1,2-a]-1,4-benzodiazepine (1.15 g) is combined with phenylisothiocyanate (395 mg, 2.93 mmole) in $CH_2Cl_2$ (20 mL) and the mixture concentrated in a steam bath. The resulting product is twice diluted with $CH_2Cl_2$ (20 mL) and both times reconcentrated on the steam bath. The product is evaporated in vacuo to a foam which is treated with TFA (15 mL) and warmed for 18 minutes in an oil bath thermostated at 52°, with the TFA being removed in vacuo. The residue is treated twice with $CH_2Cl_2$ and with $Et_2O$, evaporated in vacuo after each treatment, and the resulting product chromatographed on silica gel. The product fractions are evaporated in vacuo, and the residue is dissolved in $CH_2Cl_2$, washed with a small volume of 5% NaOH, dried over $Na_2SO_4$, filtered, and evaporated to give the 4-(S) isomer of the title structure.

4(R)-(2(S)-amino-3-phenylpropanoylamino)-6-phenyl-4H-pyrrolo[1,2-a]-1,4-benzodiazepine was converted by the same procedure to the 4-(R) enantiomer of the title compound.

## EXAMPLE 7

Preparation of 4(S)-4-(2-indolecarbonylamino)-6-phenyl-4H-pyrrolo-[1,2-a]-1,4-benzodiazepine (62, $X^1$=H, $R^2$=Ph, $R^3$=NHCO-2-indole, W=Y=Z=H

4(S)-4-Amino-6-phenyl-4H-pyrrolo[1,2-a]-1,4-benzodiazepine (595 mg) is dissolved in $CH_2Cl_2$ (15 mL) and treated with 2-indolecarbonyl chloride (403 mg, 2.24 mmole) followed by triethylamine (227 mg, 2.24 mmole). The mixture is stirred at room temperature for approximately 30 minutes and concentrated in vacuo. The residue is chromatographed on silica gel and the combined product fractions evaporated to dryness in vacuo. Three times, $Et_2O$ (15 mL) is added and evaporated in vacuo to give the title compound.

## EXAMPLE 8

4(R)-4(3-Methoxyphenylaminocarbonylamino)-6-phenyl-4H-pyrrolo[1,2-a]-1,4-benzodiazepine (61, $X^1$ = H, $R^2$ =

Ph, $R^3$ = NHCONH— 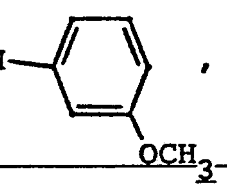 , W = Y = Z = H)

To a solution of 85 mg of 4(R)-amino-6-phenyl-4H-pyrrolo[1,2-a]-1,4-benzodiazepine in 8 ml of dry tetrahydrofuran is stirred in 3-methoxyphenylisocyanate (40 µl, 0.315 mmole) at room temperature. Stirring is continued for 8 more hours and the reaction mixture is filtered with the collected product being washed with hot methanol and dried in vacuo.

## EXAMPLE 9

Preparation of 6-phenyl-4H-imidazo[1,2-a][1,4] benzodiazepine (1, Scheme III, X = H, $R^1$ = H, $R^2$= phenyl, $R^3$ = H)

This compound is prepared according to the method of Gall and Kamdar, J. Org. Chem., 46, 1575-1585 (1981).

## EXAMPLE 10

Preparation of 4-oximino-6-phenyl-4H-imidazo[1,2-a]-1,4-benzodiazepine(58, $X^1$ = H, $R^2$ = Ph, W = Y = CH, Z = N

This compound is prepared according to the method of Example 2.

## EXAMPLE 11

4-Amino-6-phenyl-4H-imidazo[1,2-a]-1,4-benzodiazepine (59, $X^1$ = H, $R^2$ = Ph, W = Y = Ch, Z = N, n = O)

This compound is prepared according to the method of Example 3 and resolved according to the methods of Examples 4 through 6.

EXAMPLE 12

4(S)-4-(4-Chlorophenylcarbonylamino)-6-phenyl-4H-imidazo[1,2-a]-1,4-benzodiazepine (1, $X^1$ = H,

$R^2$ = phenyl, $R^1$ = H, $R^3$ = NHCO

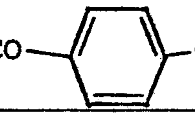

Cl)

This compound is prepared according to the method of Example 7.

EXAMPLE 13

4(R)-4-(3-Methylphenylaminocarbonylamino)-6-phenyl-4H-imidazo[1,2-a]-1,4-benzodiazepine (1, $X^1$ = H, $R^1$ = H,

$R^2$ = phenyl, $R^3$ = NHCONH

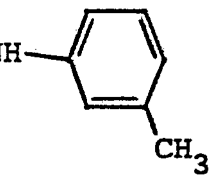

)

This compound is prepared according to the method of Example 8.

EXAMPLE 14

1-Methyl-6-phenyl-3-propyl-4H-imidazo[1,5-a]-1,4-benzodiazepine (1, Scheme V, $X^1$ = H, $R^2$ = Ph, $R^1$ = methyl, $R^3$ = H, $R^{17}$ = propyl

This compound is prepared according to the methods of Walser et al, J. Heterocyclic Chem., 15 855-858 (1978).

EXAMPLE 15

1-Methyl-4-oximino-6-phenyl-3-propyl-4H-imidazo-[1,5-a]-1,4-benzodiazepine (58, $X^1$ = H, $R^2$ = Ph, W = C-$CH_3$, Y = N, Z = C-$CH_2CH_2CH_3$)

This compound is prepared according to the method of Example 2.

EXAMPLE 16

4-Amino-1-methyl-6-phenyl-3-propyl-4H-imidazo[1,5-a]-1,4-benzodiazepine (59, $X^1$ = H, $R^2$ = Ph, W = C-$CH_3$, Y = N, Z= C-$CH_2CH_2CH_3$, n = O)

This compound is prepared according to the method of Example 3 and resolved according to the methods of Example 4 through 6.

EXAMPLE 17

4(S)-4-(2-Indolecarbonylamino)-1-methyl-6-phenyl-3-propyl-4H-imidazo[1,5-a]-1,4-benzodiazepine (1, $X^1$ = H, $R^1$ = methyl, $R^2$ = Ph, $R^3$ = NHCO-2-indole, $R^{17}$ = n-propyl

This compound is prepared according to the method of Example 7.

EXAMPLE 18

4(R)-4-(3-Chlorophenylaminocarbonylamino)-1-methyl-6-phenyl-3-propyl-4H-imidazo[1,2-a]-1,4-benzodiazepine (1, $X^1$ = H, $R^1$ = methyl, $R^2$ = Ph, $R^3$ =

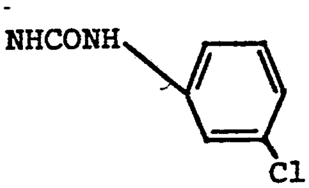

, $R^{17}$ = n-propyl)

This compound is prepared according to the method of Example 8.

EXAMPLE 19

6-Phenyl-4H-pyrazolo[1,5-a][1,4]benzodiazepine-2-carboxylic acid, ethyl ester (40, $X^1$ = H, $R^2$ = Ph, $R^{11}$ = $CH_2CH_3$

This compound is prepared according to the methods of Gilman et al, J. Heterocyclic Chem., 14, 1163 (1977).

EXAMPLE 20

4-Oximino-6-phenyl-4H-pyrazolo[1,5-a][1,4]benzodiazepine-2-carboxylic acid, ethyl ester (58, $X^1$ = H, $R^2$ = Ph, W = N, Y = $COOCH_2CH_3$, Z = CH)

This compound is prepared according to the method of Example 2.

EXAMPLE 21

4-Amino-6-phenyl-4H-pyrazolo[1,5-a][1,4]benzodiazepine-2-carboxylic acid, ethyl ester (59, $X^1$ = H, $R^2$ = Ph, W = N, Y = $COOCH_2CH_3$, Z = CH)

This compound is prepared according to the method of Example 3 and resolved according to the methods of Examples 4 through 6.

EXAMPLE 22

4(R)-4-(3-Methoxyphenylaminocarbonylamino)-6-phenyl-4H-pyrazolo[1,5-a][1,4]benzodiazepine-2-carboxylic acid, ethyl ester. (61, X = H, $R^2$ = Ph, $R^3$ =

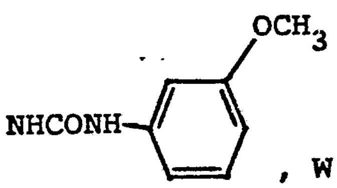

, W = N, Y = $C-COOCH_2CH_3$, Z = CH)

This compound is prepared according to the method of Example 8.

EXAMPLE 23

4-(S)-4-(2-Indolecarbonylamino)-2-methyl-6-phenyl-4H-pyrazolo[1,5-a][1,4]benzodiazepine (62, $X^1$ = H, $R^2$ = Ph, $R^3$ = NHCO-2-indole, W = N, Y = $C-CH_3$, Z= CH)

This compound is prepared according to the methods of Examples 19 through 21 and 7.

EXAMPLE 24

3-Methyl-6-phenyl-6H-triazolo[1,5-a][1,4]-benzodiazepine(48, $X^1$ = H, $R^2$ = Ph)

This compound is prepared according to the methods of Coffen et al, J. Org. Chem., 40, 894 (1975).

EXAMPLE 25

3-Methyl-4-Oximino-6-phenyl-4H-triazolo[1,5-a][1,4]-benzodiazepine (58, $X^1$=H, $R^2$=Ph, W=Y=N, Z=$C-CH_3$)

This compound is prepared according to the method of Example 2.

EXAMPLE 26

4-Amino-3-methyl-6-phenyl-4H-triazolo[1,5-a][1,4]-benzodiazepine (59, $X^1$ = H, $R^2$ = Ph, W = Y = N, Z= C-$CH_3$)

This compound is prepared according to the method of Example 3 and resolved according to the methods of Examples 4 through 6.

EXAMPLE 27

4(S)-4-(2-Indolecarbonylamino)-3-methyl-6-phenyl-4H-triazolo[1,5-a][1,4]-benzodiazepine (62, $X^1$ = H, $R^2$ = Ph, $R^3$ = NHCO-2-indole, W = Y = N, Z = C-$CH_3$)

This compound is prepared according to the method of Example 7.

EXAMPLE 28

4(R)-4-(3-Methylphenylaminocarbonylamino)-3-methyl-6-phenyl-4H-triazolo[1,5-a][1,4]-benzodiazepine (61,

$X^1$ = H, $R^2$ = Ph, $R^3$ = NHCONH 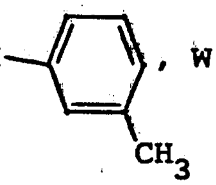 , W = Y = N,

Z = C-$CH_3$)

This compound is prepared according to the method of Example 8.

EXAMPLE 29

6-Phenyl-4H-tetrazolo[1,5-a][1,4]benzodiazepine (1, Scheme VII, X = H, $R^2$ = Ph, $R^3$ = H)

This compound is prepared according to the methods of Hester, et al, Tetrahedron Lett., 20, 1609 (1971).

EXAMPLE 30

4-Oximino-6-phenyl-4H-tetrazolo[1,5-a][1,4]benzodiazepine (58, $X^1$ = H, $R^2$ = Ph, W = Y = Z = N)

This compound is prepared according to the method of Example 2.

EXAMPLE 31

4-Amino-6-phenyl-4H-tetrazolo[1,5-a][1,4]benzodiazepine (59, $X^1$= H, $R^2$ = Ph, W = Y = Z = N)

This compound is prepared according to the method of Example 3 and resolved according to the methods of Examples 4 through 6.

EXAMPLE 32

4(S)-4-(2-Indolecarbonylamino)-6-phenyl-4H-tetrazolo-[1,5-a][1,4]benzodiazepine (1, Scheme VIII, $X^1$ = H, $R^2$ = Ph, $R^3$ = NHCO-2-indole

This compound is prepared according to the method of Example 7.

EXAMPLE 33

4(R)-4-(3-Chlorophenylaminocarbonylamino)-6-phenyl-4H-tetrazolo[1,5-a][1,4]benzodiazepine(1, Scheme VIII,

$X^1$ = H, $R^2$ = Ph, $R^3$ = NHCONH 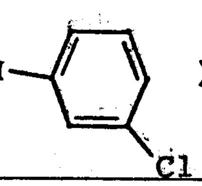 )

This compound is prepared according to the method of Example 8.

## Claims

1. A compound of the formula:

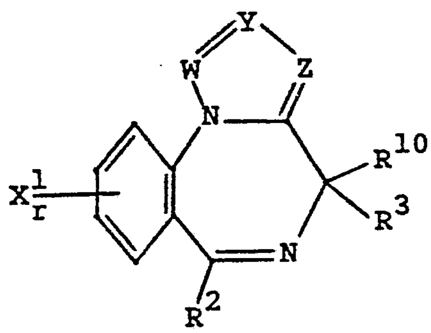

(I),

wherein

$R^1$ is H, $C_1$-$C_4$-straight- or branched-chain alkyl, cyclo-$C_3$-$C_7$-alkyl, or unsubstituted or mono- or disubstituted phenyl, where the substituent(s) is/are independently selected from the group consisting of halo, $C_1$-$C_4$-alkyl, $C_1$-$C_4$-alkoxy, and hydroxy;

$R^2$ is H; $C_1$-$C_4$-straight- or branched-chain alkyl; mono- or disubstituted or unsubstituted phenyl, where the substituent(s) is/are selected from the group consisting of halo, $C_1$-$C_4$-alkyl, $C_1$-$C_4$-alkoxy, $C_1$-$C_4$-alkylthio, carboxyl, carboxyl-$C_1$-$C_4$-alkyl, nitro, $-CF_3$, $O\overset{O}{\overset{\|}{C}}-R^4$ and hydroxy; 2-, 3- or 4-pyridyl; or $-(CH_2)_mCOOR^6$;

$R^3$ is $-(CH_2)_nR^7$,

$-(CH_2)_n\overset{OH}{\overset{|}{C}}HR^7$, $-(CH_2)_n\overset{O}{\overset{\|}{C}}R^7$,

$-(CH_2)_nNR^{18}(CH_2)_qR^7$, $-(CH_2)_nX^9\overset{O}{\overset{\|}{C}}(CH_2)_qR^7$,

$-(CH_2)_nX^9\overset{O}{\overset{\|}{C}}\underset{NHCOOR^{14}}{\underset{|}{C}}HCH_2R^7$, 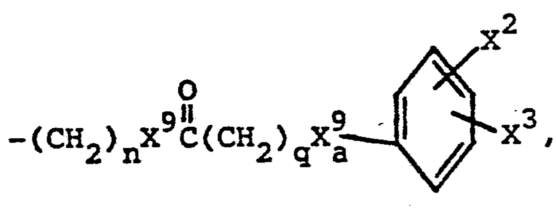

$-(CH_2)_n-X^9-\overset{O}{\overset{\|}{C}}-X^9_a-(CH_2)_q-R^7$, or

$-(CH_2)_n-X^9-\overset{O}{\overset{\|}{C}}-\overset{NH_2}{\overset{|}{C}}H-CH_2R^7$;

$R^4$ and $R^5$ are independently H, $C_1$-$C_4$-straight- or branched-chain-alkyl, cyclo-$C_3$-$C_7$-alkyl, or are connected to form a hetero

ring of the form, - $\overbrace{N(C}H_2)_k$, where k is 2 to 6;

$R^6$ is H; $C_1$-$C_4$-straight or branched-chain alkyl; cyclo-$C_3$-$C_7$-alkyl; unsubstituted or mono- or disubstituted phenyl, where the substituent(s) is/are independently selected from the group consisting of halo, $C_1$-$C_4$-alkyl, $C_1$-$C_4$-alkoxy, nitro, and $CF_3$; or unsubstituted or mono- or disubstituted phenyl-$C_1$-$C_4$-straight or branched-chain alkyl, where the substituent(s) is/are selected from the group consisting of halo, $C_1$-$C_4$-alkyl, $C_1$-$C_4$-alkoxy, nitro, and $CF_3$;

$R^7$ is α- or β-naphthyl; unsubstituted or mono- or disubstituted phenyl, where the substituent(s) is/are selected from the group consisting of halo, $-NO_2$, -OH, $-NR^4R^5$, $C_1$-$C_4$-straight- or branched-chain alkyl, cyano, phenyl, trifluoromethyl, acetylamino, acetyloxy, $C_1$-$C_4$-straight- or branched-chain alkylthio, $SCF_3$, $C\equiv CH$, $CH_2SCF_3$, $OCHF_2$, S-phenyl, or $C_1$-$C_4$-straight- or branched-chain alkoxy;

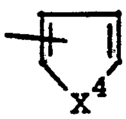 ; 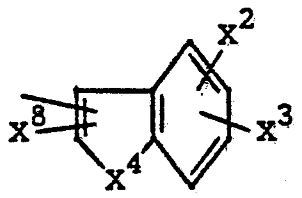 ; 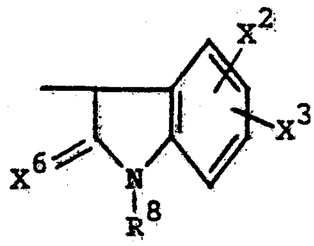 ;

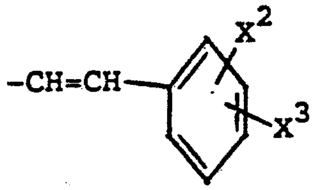 ; or 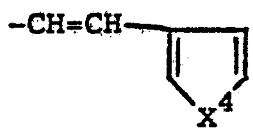 ;

$R^8$ is H, $C_1$-$C_4$-straight- or branched-chain alkyl, cyclo-$C_3$-$C_7$-alkyl, $-(CH_2)_n$-cyclo-$C_3$-$C_7$-straight- or branched-chain alkyl,

$-\overset{O}{\overset{\|}{C}}-C_1-C_4$-straight- or branched-chain alkyl, or

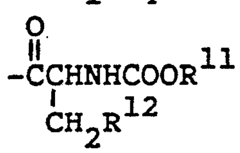 ;

$R^9$ is OH, $OR^{11}$ or 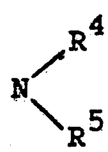 ;

$R^{10}$ is H, -OH, or $-CH_3$;

$R^{11}$ and $R^{12}$ are independently $C_1$-$C_4$-straight- or branched-chain alkyl or cyclo-$C_3$-$C_7$-alkyl;

$R^{13}$ is H or $C_1$-$C_4$-straight- or branched-chain alkyl, cyclo-$C_3$-$C_7$-alkyl,

cyclo-$C_3$-$C_7$-alkyl, $(CH_2)_n\overset{O}{\overset{\|}{C}}R^9$, $(CH_2)_mO\overset{O}{\overset{\|}{C}}R^{11}$,

$(CH_2)_mNHR^{15}$, $CH_2)_mOH$, or phenyl-$C_1$-$C_4$-straight- or branched-chain alkyl;

$R^{14}$ is $C_1$-$C_4$-straight- or branched-chain alkyl or phenyl-$C_1$-$C_4$-straight- or branched-chain alkyl;

$R^{15}$ is H or $\overset{O}{\overset{\|}{C}}$-$R^{11}$;

$R^{16}$ is H, $C_1$-$C_4$-straight- or branched-chain alkyl, cyclo-$C_3$-$C_7$-alkyl, or phenyl-$C_1$-$C_4$-straight- or branched-chain alkyl;

$R^{17}$ is H, $C_1$-$C_4$-straight- or branched-chain alkyl, $COOR^{16}$, phenyl or phenyl-$C_1$-$C_4$-straight- or branched-chain alkyl;

$R^{18}$ is H, $C_1$-$C_4$-straight- or branched-chain alkyl or formyl, acetyl, propionyl or butyryl;

m is 1-to-4;

n is 0-to-4;

q is 0-to-4;

r is 1 or 2;

$X^1$ is H, $-NO_2$, $CF_3$, CN, OH, $C_1$-$C_4$-straight- or branched-chain alkyl, halo, $C_1$-$C_4$-straight- or branched-chain alkylthio, $C_1$-$C_4$-straight- or branched-chain alkoxy,

$-(CH_2)_nCOOR^6$, $-NR^4R^5$, or O-$\overset{O}{\overset{\|}{C}}$-$R^4$;

$X^2$ and $X^3$ are independently H, -OH, $-NO_2$, halo, $C_1$-$C_4$-straight- or branched-chain alkylthio, $C_1$-$C_4$-straight- or branched-chain alkyl, $C_1$-$C_4$-straight- or branched-chain alkoxy, or

-O-$\overset{O}{\overset{\|}{C}}$-$R^4$;

$X^4$ is S, O, $CH_2$, or $NR^8$;

$X^6$ is O or HH;

$X^8$ is H or $C_1$-$C_4$-straight- or branched-chain alkyl;

$X^9$ and $X^9_a$ are independently $NR^{18}$ or O;

W = $CR^1$ or N;

Y = $CR^{13}$ or N; and

Z = N or $CR^{17}$,

or a pharmaceutically-acceptable salt thereof.

2. A compound according to Claim 1 wherein:

$R^1$ is H or $C_1$-$C_4$-alkyl; $R^2$ is unsubstituted or mono- or disubstituted phenyl, wherein the substituents are selected from the group consisting of halo, $C_1$-$C_4$-alkyl, $C_1$-$C_4$-alkoxy, nitro and $-CF_3$; $R^3$ is $-(CH_2)_nR^7$,

$$-(CH_2)_nX^9\overset{O}{\overset{\|}{C}}(CH_2)_qR^7 \text{ or } -(CH_2)_n-X^9-\overset{O}{\overset{\|}{C}}-X^9_a-(CH_2)_q-R^7;\ R^4$$

and $R^5$ are independently H, $C_1$-$C_4$-alkyl or are

connected to form the ring - $N(CH_2)_k$, where k is 4 or 5; $R^7$ is α- or β-naphthyl, unsubstituted or mono- or disubstituted phenyl, wherein the substituents are selected from the group consisting of halo, $-NO_2$, $C_1$-$C_4$-alkyl, $C_1$-$C_4$-alkoxy and trifluoromethyl

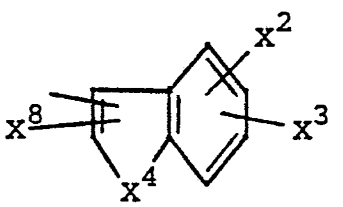 , or 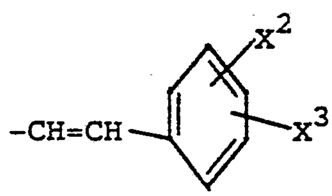 ;

$R^8$ is H or $C_1$-$C_4$-alkyl; $R^{11}$ is $C_1$-$C_4$-alkyl; $R^{13}$ is H, $C_1$-$C_4$-alkyl, cyclo-$C_3$-$C_5$-alkyl or

$$(CH_2)_n\overset{O}{\overset{\|}{C}}-R^9;$$

$R^{16}$ is $C_1$-$C_4$-alkyl; n is 0-to-2; q is 0-to-2; $X^1$ is H, $-NO_2$, $-C_1$-$C_4$-alkyl, halo or $C_1$-$C_4$-alkoxy; $X^2$ and $X^3$ are independently H, $-NO_2$, halo, $C_1$-$C_4$-alkyl or $C_1$-$C_4$-alkoxy; and $X^9$ and $X^9_a$ are independently NH or O.

3. A compound according to Claim 2, wherein

$R^1$ is H or methyl; $R^2$ is phenyl or o-F-phenyl; $R^3$ is

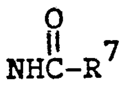 or 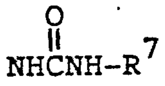; $R^7$ is 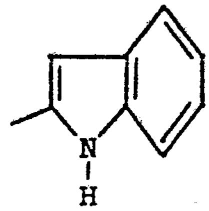, 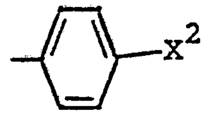,

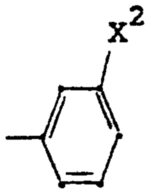, or 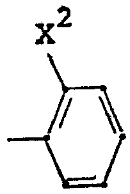;

$R^{13}$ is H, methyl, ethyl or propyl; $R^{17}$ is H, methyl or ethyl; $X^1$ is H; $X^2$ is H, $-NO_2$, halo, methyl or methoxy; W is $CR^1$; Y is $CR^{13}$ and Z is $CR^{17}$.

4. A compound according to Claim 2, wherein $R^1$ is H or methyl; $R^2$ is phenyl or o-F-phenyl; $R^3$ is

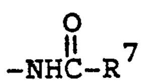 or 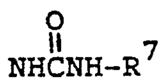; $R^7$ is 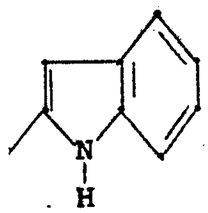, 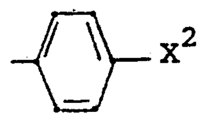,

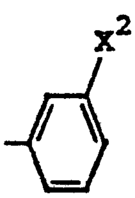, or 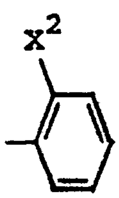; $R^{13}$ is H; $X^1$ is H;

$X^2$ is H, $-NO_2$, halo, methyl or methoxy; W is $CR^1$; Y is $CR^{13}$; and Z is N.

5. A compound according to Claim 2, wherein $R^1$ is H or methyl; $R^2$ is phenyl or o-F-phenyl; $R^3$ is

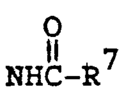 or 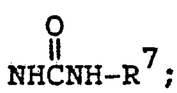; $R^7$ is 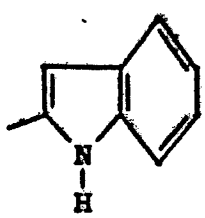, 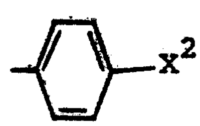,

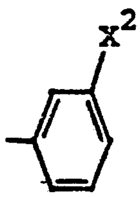 , or 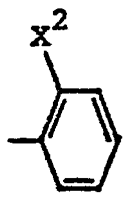 ;

$R^{17}$ is H, $CH_3$, $CH_2CH_3$, COOH, $COOCH_3$, or $COOCH_2CH_3$; $X^1$ is H; $X^2$ is H, $-NO_2$, halo, methyl, or methoxy; W is $CR^1$; Y is N and Z is $CR^{17}$.

6. A compound according to Claim 2 wherein

$R^2$ is phenyl or o-F-phenyl; $R^3$ is $NH\overset{O}{\overset{\|}{C}}$ -$R^7$ or

$NH.\overset{O}{\overset{\|}{C}}$ NH-$R^7$; $R^4$ and $R^5$ are independently H, methyl, ethyl or $-(CH_2)_4-$;

$R^7$ is 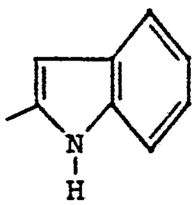 , 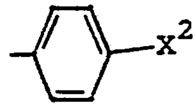 ,

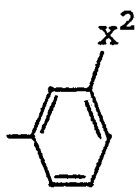 , or 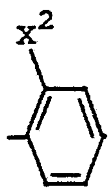 ; $R^9$ is OH, $OCH_3$,

$OCH_2CH_3$, or 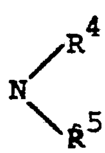 ; $R^{13}$ is methyl, ethyl, propyl or

$\overset{O}{\overset{\|}{C}}$ -$R^9$; $R^{17}$ is H, $X^1$ is H; $X^2$ is H, $-NO_2$, halo, methyl, or methoxy; W is N; Y is $CR^{13}$; and Z is $CR^{17}$.

7. A compound according to Claim 2, wherein

$R^2$ is phenyl or o-F-phenyl; $R^3$ is $NH\overset{O}{\overset{\|}{C}}$ -$R^7$ or

$NH\overset{O}{\overset{\|}{C}}NH-R^7$; $R^7$ is 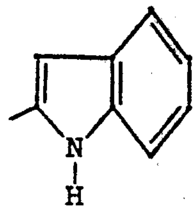, 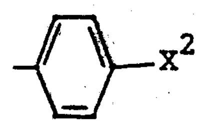,

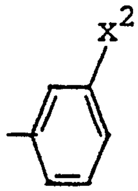, or 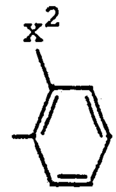; $R^{17}$ is H, methyl, $COOCH_3$,

or $COOCH_2CH_3$; $X^1$ is H; $X^2$ is H, $-NO_2$, halo, methyl or methoxy; W is N; Y is N; and Z is $CR^{17}$.

8. A compound according to Claim 2, wherein

$R^2$ is phenyl or o-F-phenyl; $R^3$ is $NH\overset{O}{\overset{\|}{C}}-R^7$ or

$NH\overset{O}{\overset{\|}{C}}NH-R^7$; $R^7$ is 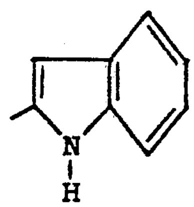, 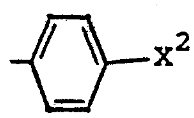,

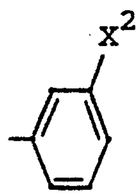, or 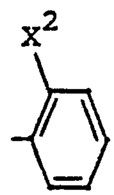;

$X^1$ is H; $X^2$ is H, $-NO_2$, halo, methyl or methoxy; W is N; Y is N; and Z is N.

9. A compound according to Claim 2, which is:
4(S)(2-indolecarbonylamino)-6-phenyl-4H-pyrrolo-[1,2-a]-1,4-benzodiazepine;
4(S)-4(4-chlorophenylcarbonylamino)-6-phenyl-4H-imidazo-[1,2-a]-1,4-benzodiazepine;
4(S)-4-(2-indolecarbonylamino)-1-methyl-6-phenyl-3-propyl-4H-imidazo[1,5-a]-1,4-benzodiazepine;
4(S)-4-(2-indolecarbonylamino)-2-methyl-6-phenyl-4H-pyrazolo[1,5-a]-1,4-benzodiazepine;
4(S)-4-(2-indolecarbonylamino)-3-methyl-6-phenyl-4H-triazolo[1,5-a]-1,4-benzodiazepine; or
4(S)-4-(2-indolecarbonylamino)-6-phenyl-4H-tetrazolo-[1,5-a]-1,4-benzodiazepine.

10. A compound according to Claim 2, which is:
4(R)-4(3-methoxyphenylaminocarbonylamino)-6-phenyl-4H-pyrrolo[1,2-a]-1,4-benzodiazepine;
4(R)-4-(3-methylphenylaminocarbonylamino)-6-phenyl-4H-imidazo-[1,2-a]-1,4-benzodiazepine;
4(R)-4-(3-chlorophenylaminocarbonylamino)-1-methyl-6-phenyl-3-propyl-4H-imidazo-[1,2-a]-1,4-benzodiazepine;
4(R)-4-(3-methoxyphenylaminocarbonylamino)-6-phenyl-4H-pyrazolo-[1,5-a]-1,4-benzodiazepine-2-carboxylic acid, ethyl ester;
4(R)-4-(3-methylaminocarbonylamino)-3-methyl-6-phenyl-4H-triazolo-[1,5-a]-1,4-benzodiazepine; or
4(R)-4-(3-chlorophenylaminocarbonylamino)-6-phenyl-4H-tetrazolo-[1,5-a]-1,4-benzodiazepine.

11. A composition comprising a therapeutically-effective amount for antagonism of the function of cholecystokinins or gastrin in mammals of one or more 1,4-benzodiazepines with 5-membered heterocyclic rings according to Claim 1, or pharmaceutically-acceptable salts thereof, and a pharmaceutically-acceptable carrier.

12. A composition according to Claim 11, further comprising an adjuvant.

13. A composition according to Claim 11, wherein the therapeutically-effective amount is about 0.05 to

50 mg/kg of body weight.

14. A composition according to Claim 11, wherein the mammals are humans.

15. The use of a 1,4-benzodiazepine with a 5-membered heterocyclic ring or a pharmaceutically-acceptable salt thereof, for the preparation of a medicament useful for preventing or treating mammals for CCK-related and/or gastrin-related disorders of the gastrointestinal, central nervous or appetite regulatory systems.

16. The use according to Claim 15, wherein the medicament also contains a pharmaceutically-acceptable carrier or a pharmaceutically-acceptable carrier and an adjuvant.

17. The use according to Claim 15, wherein the 1,4-benzodiazepines with 5-membered heterocyclic rings are selected from the compounds of the group consisting of:
4(S)-4(2-indolecarbonylamino)-6-phenyl-4H-pyrrolo[1,2-a]-1,4-benzodiazepine;
4(S)-4(4-chlorophenylcarbonylamino)-6-phenyl-4H-imidazo-[1,2-a]-1,4-benzodiazepine;
4(S)-4-(2-indolecarbonylamino)-1-methyl-6-phenyl-3-propyl-4-imidazo[1,5-a]-1,4-benzodiazepine;
4(S)-4-(2-indolecarbonylamino)-2-methyl-6-phenyl-4H-pyrazolo[1,5-a]-1,4-benzodiazepine;
4(S)-4-(2-indolecarbonylamino)-3-methyl-6-phenyl-4H-triazolo-[1,5-a]-1,4-benzodiazepine; and
4(S)-4-(2-indolecarbonylamino)-6-phenyl-4H-tetrazolo-[1,5-a]-1,4-benzodiazepine.

18. The use according to Claim 15, wherein the 1,4-benzodiazepines with 5-membered heterocyclic rings are selected from the compounds of the group consisting of:
4(R)-4(3-methoxyphenylaminocarbonylamino)-6-phenyl-4H-pyrrolo[1,2-a]-1,4-benzodiazepine;
4(R)-4-(3-methylphenylaminocarbonylamino)-6-phenyl-4H-imidazo-[1,2-a]-1,4-benzodiazepine;
4(R)-4-(3-chlorophenylaminocarbonylamino)-1-methyl-6-phenyl-3-propyl-4H-imidazo-[1,2-a]-1,4-benzodiazepine;
4(R)-4-(3-methoxyphenylaminocarbonylamino)-6-phenyl-4H-pyrazolo-[1,5-a]-1,4-benzodiazepine-2-carboxylic acid, ether ester;
4(R)-4-(3-methylphenylaminocarbonylamino)-3-methyl-6-phenyl-4H-triazolo-[1,5-a]-1,4-benzodiazepine; and
4(R)-4-(3-chlorophenylaminocarbonylamino)-6-phenyl-4H-tetrazolo-[1,5-a]-1,4-benzodiazepine.

19. The use of a compound according to Claim 1 for the preparation of a food additive useful for increasing food intake.

European Patent Office

# EUROPEAN SEARCH REPORT

Application number: EP 87311030.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| D,X | EP - A2 - 0 169 392 (MERCK & CO. INC)<br>* Claims 1,2,8-11; pages 5, 12-14 * | 1,2, 11-16 | C 07 D 487/04<br>A 61 K 31/55//<br>(C 07 D 487/04<br>C 07 D 257:00<br>C 07 D 243:00)<br>(C 07 D 487/04<br>C 07 D 249:00<br>C 07 D 243:00)<br>(C 07 D 487/04<br>C 07 D 243:00<br>C 07 D 235:00)<br>(C 07 D 487/04<br>C 07 D 243:00<br>C 07 D 231:00)<br>(C 07 D 487/04<br>C 07 D 243:00<br>C 07 D 209:00) |
| D,A | * Claims 1,2,8-11; pages 5, 12-14 *<br>-- | 3-10, 17-19 | |
| A | US - A - 3 869 450 (F.G. KATHAWALA)<br>* Claim 1 *<br>-- | 1 | |
| A | US - A - 3 862 137 (F.G. KATHAWALA)<br>* Claim 1 *<br>---- | 1 | |

TECHNICAL FIELDS SEARCHED (Int. Cl 4)

C 07 D 487/00

The present search report has been drawn up for all claims

| Place of search | Date of completion of the search | Examiner |
|---|---|---|
| VIENNA | 23-03-1988 | PETROUSEK |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82